(19) Europäisches Patentamt
European Patent Office
Office européen des brevets

(11) Publication number: 0 219 106
A2

(12) # EUROPEAN PATENT APPLICATION

(21) Application number: 86114243.8

(22) Date of filing: 15.10.86

(51) Int. Cl.⁴: C 12 N 15/00
C 07 K 13/00, C 07 H 21/04
C 12 N 1/20, A 61 K 39/21
C 12 P 21/02, G 01 N 33/569
G 01 N 33/577, C 07 K 15/00
//(C12N1/20, C12R1:19, 1:125)

(30) Priority: 17.10.85 GB 8525615

(43) Date of publication of application:
22.04.87 Bulletin 87/17

(84) Designated Contracting States:
AT BE CH DE FR GB IT LI NL SE

(71) Applicant: F. HOFFMANN-LA ROCHE & CO.
Aktiengesellschaft

CH-4002 Basel(CH)

(72) Inventor: Bannwarth, Wilhelm, Dr.
8 Liebenbergstrasse
D-7888 Rheinfelden-Beuggen(DE)

(72) Inventor: Certa, Ulrich, Dr.
21 Steinbühlweg
CH-4123 Allschwil(CH)

(72) Inventor: Mous, Jan, Dr.
15 Liebrütistrasse
CH-4303 Kaiseraugst(CH)

(72) Inventor: Stüber, Dietrich, Dr.
41 Dürerstrasse
D-7888 Rheinfelden(DE)

(74) Representative: Lederer, Franz, Dr. et al,
Patentanwält Dr. Franz Lederer Lucile Grahnstrasse 22
D-8000 München 80(DE)

(54) **Polypeptides that elicit antibodies against AIDS virus.**

(57) The present invention relates to novel polypeptides eliciting antibodies against AIDS virus and, therefore, are useful in diagnosis and therapy of AIDS, to a method of preparing synthetic genes coding for these polypeptides, to corresponding microbial expression vectors and transformants expressing these polypeptides.

EP 0 219 106 A2

./..

Croydon Printing Company Ltd

Figure 1

```
BamHI                      1                         EcoRI              2                     PstI
GATCCGAAGCTCAACAGCATCTGCTGCAACTCACTGTTTGGGGTATCAAACAGCTCCAGGCTCGAATTCTGGCTGTTGAACGTTACCTGAAAGATCAACAGCTCCTGGGTATCTGGGGCTGCAGT
    GCTTCGAGTTGTCGTAGACGACGTTGAGTGACAAACCCCATAGTTTGTCGAGGTCCGAGCTTAAGACCGACAACTTGCAATGGACTTTCTAGTTGTCGAGGACCCATAGACCCCGACGTCA

                     3                      HindIII                   4                              BamHI
GGTAAACTCATCTGCACTACTGCTGTTCCTTGGAATGCTTCTTGGTCTAATAAGCTTCTGGAACAGATCTGGAATAACATGACTTGGATGGAGTGGGACCGTGAAATCAACAATTACACTG
CCATTTGAGTAGACGTGATGACGACAAGGAACCTTACGAAGAACCAGATTATTCGAAGACCTTGTCTAGACCTTATTGTACTGAACCTACCTCACCCTGGCACTTTAGTTGTTAATGTGACCTAG
```

Basle, Suisse

RAN 4105/21 0219106

TITLE MODIFIED
see front page

# Novel Polypeptides

The present invention relates to novel polypeptides eliciting antibodies against AIDS virus and/or reacting with AIDS-antibodies, to expression vectors encoding said polypeptides, to host cells transformed with these expression vectors, to methods for producing said polypeptides by using the expression vectors and transformants and to methods for detecting the presence of AIDS antibodies in human blood. The invention also covers synthetic genes which code for said polypeptides and a process for producing such genes.

Retroviruses cause a wide variety of diseases in avian and mammalian species such as human acquired immune deficiency syndrome (AIDS) which leads to collapse of the immune system and death by a wide variety of opportunistic infections. Moreover unusual forms of cancer are associated with this syndrome. At least three retroviruses have been identified as the etiologic agent of AIDS. These viruses are the lymphadenophathy - associated virus (LAV) [Montagnier, L. et al., in "Human T-Cell Leukemia Viruses", Cold Spring Harbor Laboratory, New York, pp. 363-379 (1984)], the human T-Cell lymphotropic virus (HTLV-III) [Popovic, M. et al., Science 224, 497-500 (1984)] and the AIDS-associated retrovirus (ARV) [Levy, J.A. et al., Science 225, 840-842 (1984)].

LAV, HTLV-III and ARV genomes have been molecularly cloned [Shaw, C.M. et al., Science 226, 1165-1171 (1984);

Ar/20.6.86

Levy, J.A. et al., Science 312, 760-763 (1984); Alizon, M. et al., Nature 312, 757-760 (1984)]. The complete nucleotide sequence of the proviral genome of LAV, HTLV-III and ARV-II has been determined [Wain-Hobson, S. et al., Cell 40, 9-17 (1985); Ratner, L. et al., Nature 313, 277-284 (1985); Sanchez-Pescador, R. et al., Science 227, 484-492 (1985)].

The sequence data suggest that the LAV, HTLV III and ARV-II genomes are organized similarly to other retroviruses and contain at least (i) a gag gene that encodes the internal structural (nucleocapsid or core) proteins, (ii) a pol gene that encodes the reverse transcriptase, and (iii) an env gene that encodes the envelope glycoproteins of the virion.

The glycoproteins encoded by the env gene of retroviruses are exposed on the surface of the viral particles [Kemel, S.J. et al., Virology 55, 464-475 (1973)] and are known to be essential in the early stage of viral infection for interaction with receptors on the surface of target cells [Delarco, J. and G.J. Todaro, Cell 8, 365-371 (1976)]. In addition these glycoproteins are immunologically reactive with sera from retrovirus infected patients, demonstrating their utility for diagnosis of retrovirus infections [Kiyokawa, T. et al., Proc. Natl. Acad. Sci. USA 81, 6202-6206 (1984)].

The env gene of the HTLV-III specifies a glycosylated polypeptide (gp) with a molecular weight of 160 000 (gp 160) that is processed to gp 120 and gp 41 [Robey, W.G. et al., Science 228, 593-595 (1985)].

HTLV-III env gene products synthesized in E. coli are recognized by antibodies present in the sera of AIDS patients (Crowl, R. et al., infra; Chang, N.T. et al., Science 228, 93-96 (1985)]. This suggests that these env products will be useful for the diagnosis of HTLV-III infec- tion and possibly as a vaccine against AIDS. However, restriction enzyme mapping and sequence analysis of the env gene of different HTLV-III isolates reveal significant divergence, especially in the external portion of this protein [Hahn, B.H. et al., Proc. Natl. Acad. Sci. USA 82, 4813-4817 (1985); Crowl, R. et al., Cell 41, 979-986 (1985)]. These findings raise the possibility that HTLV-III isolates from different individuals could have important biological differences in their envelope antigens, a consideration relevant to ongoing attempts to develop a vaccine against and a sensitive method for the diagnosis of AIDS.

Therefore, the polypeptides of the present invention have been synthesized which permit to screen for, diagnose and/or protect by vaccination against the AIDS virus. AIDS virus will be understood to include all variants which have been claimed as the causative agent of AIDS, e.g. LAV, ARV and HTLV-III.

More precisely the present invention provides a polypeptide having an amino acid sequence represented by the following formula:

MRGSEAQQHLLQLTVWGIKQLQARILAVERYLKDQQLLGIWGCSGKLICTTA
VPWNASWSNKLLEQIWNNMTWMEWDREINNYTGSVDLQPSLDSC (ENV(80)) I

as well as fragments and fusion polypeptides thereof eliciting antibodies against AIDS virus (antigenic polypeptides) and/or reacting with AIDS-antibodies (immunogenic polypeptides). In addition, the invention provides antigenic and/or immunogenic polypeptides related to any of the foregoing polypeptides by amino acid substitutions. The invention also encompasses tandem repeats, e.g. from 2 to 10 times, of the above mentioned immunogenic and/or antigenic polypeptides. The invention further provides processes for the manufacture of the above said immunogenic and/or antigenic polypeptides.

The polypeptides of the present invention, due to the methods for their production, may contain methionine (for which ATG codes) as amino terminal first amino acid. On the other hand the microbial host may process (completely or partly) the translation product to delete the amino terminal methionine.

In the present invention fragments are defined as selected regions of the intact polypeptide. Suitable fragments can e.g. be selected from the carboxy-terminus or from the amino-terminus of the intact polypeptide. Preferred fragments which can be used are those encoded by the DNA inserts of subclones pA1 and pA4, respectively, which are described in detail in example 2. A most preferred fragment is the polypeptide of formula

EAQQHLLQLTVWGIKQLQARILAVERYLKDQQLLGIWGCSGKLICTTA
VPWNASWSNKLLEQIWNNMTWMEWDREINNYT II

Amino acid substitutions in polypeptides which do not essentially alter their biological activities are known in the art and described, e.g. by H. Neurath and R.L. Hill in "The Proteins", Academic Press, New York (1979), in particular in fig. 6 of page 14. The most frequently observed amino acid substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and vice versa.

The fusion polypeptides of the present invention are composed of an immunogenic and/or antigenic polypeptide as defined above and a carrier protein that can advantageously be used in the purification of this polypeptide, e.g. by affinity chromatography. The preferred carrier proteins used in this invention are E. coli chloramphenicol acetyltransferase (CAT) and dihydrofolate reductase (DHFR), especially mouse DHFR.

The invention further provides synthetic genes coding for the polypeptides of the present invention, expression vectors containing such genes, and transformants useful in the production through recombinant DNA technology of the polypeptides of the invention as well as processes for the manufacture of such synthetic genes, expression vectors and transformants. Another object of the present invention is the isolation and use of the resulting polypeptides.

The present invention provides in addition a diagnostic method for testing human blood for the presence of antibodies to the AIDS virus and a test kit useful in this method. This diagnostic method overcomes the problem of non-specificity of all previously used blood tests for AIDS.

However, the polypeptides of the present invention cannot only be used as diagnostic tools but also as therapeutic agents. Therefore, a further object of the present invention is the use of these polypeptides for (a) providing protective immunity against AIDS virus when incorporated into a vaccine and (b) raising poly- and/or monoclonal antibodies against AIDS polypeptides as well as such vaccines and antibodies themselves.

The polypeptides of the present invention may be produced by conventionally known methods. The processes by which the novel protein may be produced can be divided into three groups: (1) chemical synthesis, preferably solid-phase synthesis; (2) preparation of a corresponding gene prepared by chemical synthesis which is inserted into a host and expression of the protein by the host; and (3) a corresponding gene obtained from a virus is inserted into a host and the protein is expressed by the host.

In a preferred embodiment the present invention can be described as follows:

Oligonucleotide fragments are synthesized which, when assembled correctly, form a synthetic gene coding for a polypeptide defined above. The fragments can be hybridized and ligated in pre-determined stages to construct the synthetic gene. The synthetic gene can be provided with suitable restriction enzyme sites and cloned into vectors specifically designed to maximise expression of the synthetic gene. A polypeptide having immunogenic activity to AIDS virus can thus be expressed.

The oligonucleotide fragments can be obtained in accordance with methods well-known in DNA chemistry including total chemical synthesis of the oligonucleotide sequence, e.g. in a nucleotide synthetizer.

In order to construct said synthetic gene which codes for the desired polypeptide several criteria should be observed. Firstly trinucleotide codons should be used which are acceptable to or preferably used in the cells, in particular E. coli. Secondly, it would be desirable to have restriction enzyme recognition sites at the termini of the molecule so as to allow insertion into a vector of plasmid or phage origin. Moreover, restriction sites should be selected which allow the use of well-understood cloning and expression vectors, such as plasmids of the pDS-family. Thirdly, a series of restriction endonuclease recognition sites strategically placed along the molecule should be introduced to exchange and modify parts of the gene very easily and to allow the expression or certain parts of it. Fourthly, the synthesis should not be unnecessarily complicated and illegimitate cross-hybridisations should be minimised in order to facilitate gene assembly.

Bearing in mind the above mentioned considerations a synthetic gene, designated env(80), was synthesized which is described in Fig. 1. The gene can be assembled from 22 synthetic oligonucleotide fragments (19mers up to 29mers) by single-strand overlaps to give the complete double--stranded nucleotide sequence. More details of the synthesis of the oligonucleotide fragments and the assembly of the synthetic gene are given in Examples 1 and 2.

The synthetic gene of the present invention can be introduced in any convenient expression vector of plasmid or phage origin in a manner known per se. Convenient expression vectors of plasmid or phage origin are mentioned e.g., in the laboratory manual "Molecular Cloning" by Maniatis et al., Cold Spring Harbor Laboratory, 1982.

Plasmids of the pDS family are specific examples of plasmid expression vectors of the present invention. These plasmids are derivatives of pBR 322 and comprise e.g., pDS5/RBS II, 3A+5A; pDS6/RBS II, 3A+5A and pDS8/RBS II resulting in plasmid constructions like pENV(80), pENV(80)/CAT and pENV(80)/DHFR containing the synthetic gene operatively linked to a coliphage T5 promoter/lac operator expression control sequence.

E. coli strains containing plasmids useful for such constructions (E. coli M15 transformed with pDS5/RBS II, 3A+5A; pDS6/RBS II, 3A+5A; pDS8/RBS II) have been deposited at the Deutsche Sammlung von Mikroorganismen (DSM) in Göttingen on october 3, 1985, the accession nos. being DSM 3517, DSM 3518, DSM 3519, respectively.

It should, of course, be understood that the synthetic gene inserted at a selected site of the expression vector may be fused to DNA sequences which do not code for ENV(80) or may code only for a fragment of ENV(80). It is only required that the inserted DNA sequence (synthetic gene), in a transformed host, will produce a polypeptide (ENV(80), fragments or fusion polypeptides thereof or analogs obtained by amino acid substitution(s)) having an immunogenic activity to the AIDS virus.

The DNA sequences that may be fused to the synthetic gene may be selected from a large variety of DNA sequences that encode prokaryotic or eukaryotic polypeptides. Preferred DNA sequences used to fuse to the synthetic gene in the present invention are those coding for dihydrofolate reductase (DHFR), especially mouse DHFR, and E. coli chloramphenicol acetyltransferase (CAT).

Methods for expressing DNA sequences coding for the polypeptides of this invention are well known, e.g. from Maniatis et al., supra. They include transforming an appropriate bacterial host with an expression vector having the said DNA sequence operatively linked to the expression control sequence of the vector, culturing the host under appropriate conditions of growth and extracting and isolating the desired polypeptide from the culture. Those of skill in the art may select from these known methods those that are most effective for a particular gene expression without departing from the scope of this invention.

The selection of a particular host for use in this invention is dependent upon a number of factors recognized by the art. These include, for example, compatibility with the chosen expression vector, toxicity of the proteins encoded for by the hybrid plasmid, ease of recovery of the desired protein, expression characteristics, biosafety and costs. Within these general guidelines, examples of useful bacterial hosts are gram-negative and gram-positive bacteria, especially strains of E. coli and B. subtilis. The most preferred host cell of this invention is E. coli M15 (described as DZ291 in M.R. Villarejo et al., "ß-Galactosidase from termination and deletion mutant strains", J. Bacteriol., 120, pp. 466-474 [1974]). However, other E. coli strains, such as E. coli 294 (ATCC No. 31446) and E. coli RR1 (ATCC No. 31343), can also be used.

The polypeptides of the present invention can be purified by known methods, such as precipitation with ammonium sulfate, dialysis to remove salts (under normal or reduced pressure), gel filtration, chromatography, preparative flat-bed iso-electric focusing, gel electrophoresis, high performance liquid chromatography (hereinafter HPLC; including ion exchange, gel filtration and reverse phase chromatography), and affinity chromatography, e.g. on dye bound carrier or on Sepharose coupled with monoclonal antibodies against said polypeptide, and the like. In a preferred embodiment of the invention the fusion polypeptides of the present invention are purified by affinity chromatography using chloramphenicol-columns (CAT-fusion proteins) or methotrexate-columns (DHFR-fusion proteins).

The polypeptides of the present invention can be used as a vaccine capable of inducing protective immunity against the AIDS virus. Routes of administration, antigen doses, number and frequency of injections will vary from individual to individual and may parallel those currently being used in providing immunity in other viral infections. The vaccines can be prepared in accordance with known methods. The vaccine compositions will be conveniently combined with physiologically acceptable carrier materials. The vaccine compositions may contain adjuvants or any other enhancer of immune response. Furthermore, the vaccine compositions may comprise other antigens to provide immunity against other diseases in addition to AIDS.

In addition the polypeptides of the present invention may be used as diagnostic reagents for the detection of AIDS-associated antibodies in accordance with methods well-known in the art. The main advantage of the present polypeptides in the determination of antibodies against AIDS consists in their specificity when compared with known antigens used so far.

According to one method for the determination of antibodies against AIDS virus the so-called "Western Blotting" analysis is used [Tobin, H., Staehelin, Th. and Gordon, J., Proc. Nat. Acad. Sci. USA 76, 4350-4354 (1979)]. According to this technique a polypeptide of the present invention is transferred from the SDS-polyacrylamide gel electrophoretically on to nitrocellulose paper. This nitrocellulose paper is then treated with the serum to be tested. After washing, the nitrocellulose paper is then treated with an anti-human IgG labeled with peroxidase. The peroxidase is then determined by a suitable substrate, e.g. with o-phenylene diamine. Of course other labels like radioactive or fluorescence labels may be used.

A more convenient technique for the determination of antibodies against AIDS virus using a polypeptide of the present invention is an enzyme-linked immunosorbant assay (ELISA). According to this test a polypeptide of the present invention is adsorbed to the wells of a microtiterplate. The wells are then treated with sera to be tested. After washing, anti-human IgG labeled with peroxidase is added to the wells. The determination of the peroxidase is performed with a corresponding substrate, e.g. with o-phenylene diamine. Also in this procedure the peroxidase can be exchanged by another label, e.g. by a radioactive or fluorescence label.

Just another method for the determination of antibodies against AIDS virus with the polypeptides of the invention is an enzyme immunological test according to the so-called "Double-Antigen-Sandwich-Method". This method is based on the work of Maiolini, R.I., as described in Immunological Methods 20, 25-34 (1978). According to this method the serum to be tested is contacted with a solid phase on which a polypeptide of the present invention is coated and with a polypeptide of the present invention which is labeled with peroxidase. The immunological reaction can be performed in one or in two steps. If the immunological reaction is performed in two steps then a washing step is performed between the two incubations. After the immunological reaction or reactions a washing step is performed. Thereafter the peroxidase is determined with a substrate, e.g. with o-phenylene diamine.

Suitable solid phases are organic and inorganic polymers [amylases, dextrans, natural or modified celluloses, polyacrylamides, agaroses, magnetite, porous glass powder, polyvinylidene fluoride (Kynar) and latex], the inner wall of test vessels (test tube, titer plates or cuvettes of glass or artificial material) as well as the surface of solid bodies (rods of glass and artificial material, rods with terminal thickening, rods with terminal lobes or lamellae). Spheres of glass and artificial material are especially suitable solid phase carriers.

The polypeptides of the present invention are not only useful in the determination of antibodies against AIDS virus, but also for the determination of the AIDS virus itself since these polypeptides are useful in eliciting antibodies, in particular monoclonal antibodies, against AIDS virus. Such antibodies can be produced by injecting a mammalian or avian animal with a sufficient amount of a polypeptide of the present invention and recovering said antibodies from the serum of said animals.

Suitable host animals for eliciting antibodies include mammals such as rabbits, horses, goats, guinea-pigs, rats, mice, cows, sheep, etc.

Various methods which are generally known can be employed in the determination of AIDS virus.

In one such procedure known amounts of a serum sample to be assayed, radiolabeled polypeptide of the present invention and unlabeled polypeptide of the present invention are mixed together and allowed to stand. The antibody/antigen complex is separated from the unbound reagents by procedure known in the art, i.e. by treatment with ammonium sulphate, polyethylene glycol, second antibody either in access or bound to an insoluble support, dextran-coated charcoal and the like. The concentration of the labeled polypeptide of the present invention is determined in either the bound or unbound phase and the AIDS content of the sample can then be determined by comparing the level of labeled component observed to a standard curve in a manner known per se.

Another suitable method is the "Double-Antibody-Sandwich-Assay". According to this assay the sample to be tested is treated with two different antibodies. One of these antibodies is labeled and the other is coated on a solid phase. As solid phases those mentioned earlier in this application come into consideration. Suitable labels are enzymes, e.g. peroxidase, radio-labels or fluorescence-labels.

The preferred solid phase is a plastic bead and the preferred label is horse-radish peroxidase.

Different antibodies can e.g. be achieved by immunizing different animals, e.g. sheep and rabbits.

Another method consists in using the well-known Koehler and Milstein technique for producing monoclonal antibodies. In order to find out different monoclonal antibodies which are directed against the same antigen, but against different epitopes, the method of Stähli et al. [J. of Immunological Methods 32, 297-304 (1980)] can be used.

Of course, it is also possible to use an antiserum (polyclonal antibody) and a monoclonal antibody.

According to the "Double-Antibody-Sandwich-Method" the sample is incubated with the solid phase antibody and the labeled antibody. It is possible to treat the sample first with the solid phase antibody and after washing to treat the sample with the labeled antibody. However, it is also possible to treat the sample first with the solid phase antibody and after a certain time with the labeled antibody. In addition and preferably it is possible to treat the sample together with the solid phase and the labeled antibody.

After the immunological reaction(s) there is performed a washing step. After washing the label is determined according to procedures known in the art. In case that peroxidase is used as label the determination is performed with the substrate, e.g. with o-phenylene diamine or with tetramethyl benzydine. The amount of the labeled component is proportional to the amount of the antigen(s) present in the sample.

The methods for the determination of AIDS virus or of antibodies against AIDS virus as described above can be conducted in suitable test kits comprising in a container a polypeptide of the present invention or antibodies against AIDS virus elicited by a polypeptide of the present invention.

The present invention will be better understood on the basis of the following examples in connection with the following figures:

The following abbreviations and symbols used are:
A,B,E,H,P,S,X and Xb which indicate sites for restriction endonucleases AatII, BamHI, EcoRI, HindIII, PstI, SalI, XhoI and XbaI, respectively. represents promoters of the genes bla, lacI and neo; represents ribosomal binding sites of the genes bla, cat, neo and lacI; represents terminators $t_o$ and T1; represents the regulatable promoter/operator element $P_{N25*/0}$; represents ribosomal binding sites RBSII and RBSII,3A+5A; represents coding regions under control of these ribosomal binding sites; represents regions required for DNA replication (repl.); represents coding regions for dihydrofolate reductase (dhfr), chloramphenicol acetyltransferase (cat), ß-lactamase (bla), lac repressor (lacI) and neomycin phosphotransferase (neo); represents env(80)-gene.

Figure 1 is a representation of the synthetic oligonucleotide fragments constituting the synthetic gene.

Figure 2 is a schematic outline of the construction of plasmid pA-ENV-20. In the upper left part of the figure the assembly of the synthetic oligonucleotides to four units and the subcloning of these gene-blocks in pUC-plasmids is shown. The lower left and the right part of the figure display the assembly of these blocks resulting in plasmid pA-ENV-20 containing the env(80)-gene as an BamHI-fragment.

Figure 3 Part a) is a schematic drawing of the plasmid pDS5/RBSII,3A+5A. The nucleotide sequence of the XhoI/XbaI fragment containing the regulatable promoter/operator element $P_{N25*/0}$, the ribosomal binding site RBSII,3A+5A, the cat-gene and terminator T1 is displayed in part b). Here the restriction endonuclease sites indicated in part a) are overlined and the region under control of RBSII,3A+5A, encoding chloramphenicol acetyltransferase with 21 additional amino acids at its N-terminus (CAT*), is underlined. In addition the pBR322 entity of pDS5/RBSII,3A+5A is schematically shown, where the given numbers refer to the nucleotide sequence of pBR322 [J.G. Sutcliffe, Cold Spring Harbor Symp. Quant. Biol, 43, pp. 77-90 (1979)].

Figure 4 Part a) is a schematic drawing of the plasmid pDS6/RBSII,3A+5A. The nucleotide sequence of the XhoI/XbaI fragment containing the regulatable promoter/operator element $P_{N25*/O}$, the ribosomal binding site RBSII,3A+5A, terminator $t_o$, the cat-gene and terminator T1 is displayed in part b). Here the restriction endonuclease sites indicated in part a) are overlined and the coding region under control of RBSII,3A+5A is underlined. In addition the pBR322 entity of pDS6/RBSII,3A+5A is schematically shown, where the given numbers refer to the nucleotide sequence of pBR322 [J.G. Sutcliffe, supra].

Figure 5 Part a) is a schematic drawing of the plasmid pDS8/RBSII. The nucleotide sequence of the XhoI/XbaI fragment containing the regulatable promoter/operator element $P_{N25*/O}$, the ribosomal binding site RBSII, the dhfr-gene, terminator $t_o$, the cat-gene and terminator T1 is displayed in part b). Here the restriction endonuclease sites indicated in part a) are overlined and the region under control of RBSII, encoding dihydrofolate reductase with 6 additional amino acids at its N-terminus (DHFR*), is underlined. In addition the pBR322 entity of pDS8/RBSII is schematically shown, where the given numbers refer to the nucleotide sequence of pBR322 [J.G. Sutcliffe, supra].

Figure 6 Part a) is a schematic drawing of the plasmid pDMI,1. The entire DNA sequence of this plasmid is displayed in parts b) and c), where the restriction endonuclease sites indicated in part a) are overlined and the coding regions for neomycin phosphotransferase (neo) and lac repressor (lacI) are underlined.

Figure 7 is a schematic outline of the construction of plasmids pENV(80)-CAT, pENV(80) and pENV(80)-DHFR. Coding regions for proteins ENV(80)-CAT, ENV-80 and ENV(80)-DHFR are indicated by arrows.

Figure 8 displays the complete amino acid sequence of protein ENV(80), whereas for protein ENV(80)-CAT and ENV(80)-DHFR only the first 100 amino acids are shown (for the complete sequences see figures 3, 5 and 7).

Figure 9 displays the recognition of ENV(80)-CAT, ENV(80)-DHFR and ENV(80) by antibodies of an AIDS patient. Part a) is an electropherogram monitoring protein synthesis in E.coli M15 harboring pDMI,1 and in addition plasmids pDS5/RBSII,3A+5A (lane 1), pENV(80)-CAT (lane 2, pDS8/RBSII (lane 3), pENV(80)-DHFR (lanes 4), pDS6/RBSII,3A+5A (lane 5) or pENV(80) (lane 6) under uninduced or induced conditions (see Example 5). Part b) shows the corresponding immunoblot. M denotes a protein size marker, the molecular weights are indicated.

Figure 10 shows the device for the simultaneous oligonucleotide synthesis.

The construction and expression of the synthetic gene, designated env(80), of this invention is described in more detail in the following examples.

## Example 1

Synthesis of the synthetic oligonucleotide fragments constituting the synthetic gene

The synthetic oligonucleotide fragments are shown in Fig. 1 of the accompanying drawings. The oligonucleotide fragments themselves were prepared simultaneously using controlled pore glass (CPG) as support material [B.S. Sproat and W. Bannwarth, Tetrahedr. Lett., 24, 5771-5774 (1983); Adams, S.Z. et al., J. Amer. Chem. Soc., 105, 661-663 (1983)]. The device which was constructed for this purpose is shown in Fig. 10. It consists in principle of a number of disks stacked on a central shaft, so that each disk can be rotated individually. Apart from the hole for the central shaft, each disk contains four small holes and a larger one situated 72° from each other. The larger one represents the reaction chamber with frits at the bottom and top of the reaction chamber. Disk 1 and 14 are metal plates to ensure a symmetrical pressure delivered from the spring loaded nut. Disks 2 and 13 contain no reaction chambers and serve for the fitting of the tubing for the inlets and outlets of solvents and reagents.

Before a simultaneous synthesis is being started, the appropriate amount of functionalized support carrying the corresponding 3'-nucleoside of the DNA fragment to be synthesized is loaded into each chamber. All disks then have to be turned in their respective positions corresponding to the elongation unit to be hooked onto the functionalized support, i.e. the reaction chambers needing an A as the next base are turned into position A (disk 3, 4, 7 and 10 in Fig. 10a), those which need a C to position C (disk 8 in Fig. 10a), and so on. In all the possible positions the small holes together with the reaction chambers keep up a set of a column-like system throughout the pile of disks.

One now injects into each of the 4 positions corresponding to the four different elongation units the appropriate amount of either A, T, G or C and all the sequences are simultaneously elongated. All reactions and washings during one elongation cycle are likewise performed simultaneously. Leakage is avoided by a rubber ring beneath each hole in the disks (see Fig. 10c).

For the next elongation cycle the disks have to be turned into their new positions. This can be done by unscrewing the spring loaded nut (Fig. 10a) and tightening it again after positioning of the disks. Whenever the synthesis in one of the disks is completed, its reaction chamber is turned into the empty position thus allowing the synthesis to be continued in all the other disks. Since there is virtually no back pressure, the number of disks is not limited.

The CPG-support used for the synthesis of the DNA fragments was functionalized by coupling directly the 3'-succinylated nucleoside to the support using mesitylsulfonyl-nitrotriazole (MSNT) or -chloride (MSCL) and N-methyl-imidazole. This functionalization procedure is very fast and efficient, giving loadings between 25 and 33 µmol per g of CPG-support. Unreacted aminofunctions were capped by acylation.

A. <u>Functionalization of the CPG support using MSNT or MSCL and N-methylimidazole</u>

5 g of controlled pore glass material manufactured by Pierce bearing a long chain alkylamine function and 2.5 mmol succinylated nucleoside were taken up in 50 ml anhydrous pyridine and 2 ml triethylamine and the solvents thereafter evaporated. This process was repeated and the residue taken up in 30 ml anhydrous pyridine and 12.0 mmol (3.6 g) MSNT and 1.4 ml N-methyl-imidazole were added. The suspension was reacted with occasional shaking for 1 h at room temperature.

Then the mixture was filtered through a glass sinter funnel and the support washed with pyridine and ether and dried in vacuum. The support was transferred to another flask and unreacted aminofunctions capped by addition of 15 ml of a 6.5% (w/v) solution of dimethylaminopyridine (DMAP) in THF and 15 ml of acetic acid anhydride/lutidine (1/1;v/v). After a reaction time of one hour the mixture was filtered again and the support washed with pyridine and ether and dried in vacuum. The loading was estimated by the UV-determination of the released dimethoxytrityl cation, and was usually in a range of 25-33 µmoles/g support. The MSNT can be replaced by MSCL, giving the same range of functionalization.

Each synthesis of a certain DNA-fragment was started with 1 µmol of functionalised CPG-support. As elongation units deoxynucleoside-3'-ß-cyanoethyl -N,N-diisopropylamino-amidites [Sinha, N.D. et al., Nucleic Acids Res. 12, 4539-4557 (1984)] were used which were activated for the condensation step by tetrazole. The elongation cycle for the addition of one unit is shown in the following table:

| Step | Reagent or Solvent | Purpose | Time (min) |
|---|---|---|---|
| 1) | 3% dichloroacetic acid in dichloroethane | detritylation | 5 |
| 2) | acetonitrile | wash | 4 |
| 3) | activated amidites | elongation | 5 |
| 4) | acetonitrile | wash | 0.5 |
| 5) | $Ac_2O$/lutidine DMAP/THF | capping | 3 |
| 6) | acetonitrile | wash | 0.5 |
| 7) | $I_2$-solution | oxidation | 3 |
| 8) | acetonitrile | wash | 2 |

The detritylation step was performed with dichloroacetic acid [Adams, S.Z. et al., supra] which is quite safe as far as depurination is concerned and for which the cleavage time is not dependent on the chain length, as is the case with $ZnBr_2$ as cleavage reagent. All the washing steps are performed with acetonitrile. For each elongation of a sequence (in one disk) 200 µl of a solution containing 20 mg amidite and 7 mg tetrazole in anhydrous acetonitrile were used. To ensure a complete reaction, the times for the different reactions in the elongation cycle were slightly longer than probably necessary. All solvents and reagents are delivered by a small overpressure of argon (0.02 bar) and the system works in a continous flow-like manner. The activated elongation units were taken from a stock solution prepared before the start of the simultaneous synthesis. They were injected slowly by syringe into the four channels corresponding to the four bases thereby replacing the anhydrous acetonitrile. The capping step after each elongation step is performed with a mixture of acetic acid anhydride/lutidine/THF (1/1/8;v/v) and 6.5% dimethylaminopyridine (DMAP) in THF which was mixed in a ratio of 1/2 (v/v). The oxidation step is performed after each elongation with a 0.2 M solution of iodine in THF/lutidine/$H_2O$ (78/20/2;v/v).

B. <u>Working up, purification and isolation</u>

After having finished the synthesis of all the sequences in the different disks, the support was removed and individual sequences were simultaneously worked up. In 1.5 ml Eppendorf tubes with screw caps, the support was treated with conc. ammonia to cleave off the phosphorus protecting group as well as the base-protecting group and to remove it from the support.

For this purpose, the tubes were closed and kept in an Eppendorf heating block at 56° overnight. After cooling, a small hole was pierced through the bottom of each of the Eppendorf tubes, the tubes were closed again and the resulting overpressure transferred the ammonia solutions free from support material into other Eppendorf tubes. After evaporation in a speed vac concentrator they were treated with 80% acetic acid in order to remove the DMT group. They were evaporated once more (vacuum concentrator) and part of each residue was purified on a preparative 20% polyacrylamide gel. The bands were visualized by UV-shadowing, extracted and desalted together on small reversed phase (RP)-columns (J.T. Baker Chem. Corp, Phillipsburg, N.J.). After measuring the actual amount of each DNA-fragment its purity was checked after labelling with $^{32}P$ on an analytical polyacrylamide gel.

1/10 of the total amount of CPG of each synthesis, carrying the corresponding protected oligonucleotide fragment, was placed in a 1.5 ml Eppendorf tube with a screw cap. 700 µl ammonia (min. 25%) were added, the tube was closed by the screw cap and kept for 16 hrs (overnight) in an Eppendorf heating block at 56°. Afterwards the tube was cooled down and a small hole was pierced through the bottom of the tube. It was closed and the resulting overpressure was used to transfer the ammonia solution into another tube were it was collected. The support was washed with 200 µl ammonia and the combined ammonia solutions were evaporated in the speed vac concentrator after cooling for 5 min in dry ice. The residue was taken up in 300 µl 80% acetic acid and kept for 90 min at room temperature. Approximately 900 µl ether was then added and the resulting precipitate collected by centrifugation at room temperature. The supernatant was removed, the pellet dissolved in water and part of this solution applied, after addition of urea dye and a brief heating to 95°, to a polyacrylamide gel (40 x 20 x 0.2 cm).

After electrophoresis, the bands were visualized by UV-shadowing, cut out of the gel and the DNA fragments eluted with water and desalted simultaneously using small RP-columns (J.T. Baker Chem. Corp.). The resulting amounts of the different oligonucleotide fragments were measured by UV-absorption and the purity was confirmed after labelling with $^{32}P$ on an analytical polyacrylamide gel.

## Example 2

## Construction of pA-ENV-20

### A. Principles

The synthetic oligonucleotides were subdivided into 4 gene blocks with unique restriction sites at both ends. These blocks can be subcloned in pUC-plasmids. The subclones can be assembled in a relatively simple way by using the unique AatII restriction site in the plasmid which allows the construction of hybrid plasmids between the subclones. The strategy is outlined schematically in figure 2.

### B. Ligation of the synthetic oligonucleotides to individual "gene blocks"

The lyophylised oligonucleotides were dissolved in water for 1 hour at room temperature to give a DNA concentration of 10 nmole/ml. 100 pmoles of each oligonucletide were kinased with 1µl of $\gamma$ $^{[32P]}$-ATP (2 pmoles; 5000 Ci/mMol), 0.5 U T4-Polynucleotide kinase (BRL, Basel) in 50µl 50 mM TRIS-HCl, pH 8.0, 10 mM $MgCl_2$ for 5 min at 37°C. Each reaction except for the end fragments of each gene block was chased by the addition of 6µl of 0.5 mM ATP. The reaction was stopped by heating the samples for 5 min at 65°C. 10µl of each reaction mixture were aliquoted into 4 Eppendorf--tubes corresponding to the gene blocks. After adding 5µl of 1M TRIS-HCl, pH 7.8, 1µl 1M $MgCl_2$, 2µl 5M NaCl and 32µl $H_2O$ the samples were boiled for 5 min. The tubes were then placed in a beaker containing 2 liters of boiling water. The samples were cooled slowly to 4°C by placing the beaker in the cold room for about 4 hours. Ligation was carried out for 24 hours at 12°C by adding 10µl of 1M DTT

(dithiothreitol), 1µl of 100 mM ATP and 5µl T4-DNA--ligase (5 Weiss-units, Pharmacia, Uppsala). The samples were then precipitated on dry ice for 10 min by adding 12µl of 5M lithiumacetate and 260µl isopropanol. The tubes were centrifuged for 10 min at 12.000 rpm in a microfuge. The supernatant was withdrawn with a pasteur-pipette and the pellets were washed with 1 ml of 80% ethanol prior to drying in vacuo. The DNA pellets were dissolved in 10µl gel sample buffer (0.05% bromophenol blue, 0.05% xylencyanol, 10 mM EDTA (ethylendiamine-tetraacetic acid), pH 8.0). The samples were then electrophoresed on a 10% polyacrylamide gel (50x50x1mm) containing 1xTBE [0.089M TRIS--borate, 0.089M boric acid, 0.002M EDTA, T. Maniatis et al., Molecular cloning, Cold Spring Harbor (1982)] for 20 min at 400 V in a Biorad-mini-slab gel system. After electrophoresis the gel was stained for 5 min in ethidiumbromide (10µg/ml). The DNA bands were visualised under 300 nm UV light. Bands of the appropriate size were cut out using a scalpell. Marker DNA was phage ΦX digested with HAE III (BRL, Basel). The gel pieces were transferred in 4x4 mm wells in an 0.7% agarose gel and the wells were sealed with liquid 0.7% agarose in 1xTBE in order to give a homogenous electrical field. In front of each sample a piece of NA45 membrane (Schleicher and Schuell, Dassel) was inserted and the DNA was electrophoresed on the membrane for 5 min at 300 V. The strips with the DNA were then washed with distilled water and transferrred into an Eppendorf tube containing 250µl of 1.5 M lithiumacetate, 50 mM TRIS-HCl pH 8.0 and 10 mM EDTA. The DNA was eluted for 20 min at 65°C with occasional vortexing. The membrane strips were removed from the tubes and the samples were once extracted with 200µl phenol that was saturated with 1M TRIS pH 8.0. After spinning the samples for 10 min at 12.000 rpm in the microfuge the supernatant was withdrawn. The DNA was precipitated on dry ice for 10 min after adding 20µl of 5M lithiumacetate and 440µl isopropanol. The DNA was pelleted for 10 min at 12.000 rpm in a microfuge. The pellets were washed with 80% ethanol and dried in vacuo. The pellets were dissolved in 10µl of water.

C. Subcloning of the synthetic gene fragments in pUC vectors

The plasmids pUC18 and pUC19 were obtained from Pharmacia (Uppsala) with a DNA concentration of 0.4µg/µl. 2µl plasmid DNA were digested with 10 U of the appropriate enzyme in 1xT4-polymerase buffer [T. Maniatis et al., supra] for 1 h at 37°C in a total volume of 50µl. pUC18 was digested with BamRI and EcoRI for subclone I and with BamRI and HindIII for subclone 4. pUC19 was digested with EcoRI and PstI for subclone 3 and with PstI and HindIII for subclone 3 (see figure 2). After digestion the DNA was once phenol extracted, precipitated and dried as described above. The pellets were dissolved in 10µl gel loading buffer and electrophoresed on a 0.7% agarose gel containing 1xTBE and 1µg/ml ethidiumbromide. The vector bands were visualised under 300 nm UV-light. A slit was then cut in front of each band and a piece of NA45 membrane was inserted. The DNA was electrophoresed on the membrane at 300 V for 10 min. The DNA was eluted and purified as described above. The final pellet was dissolved in 30µl water to give a final DNA concentration of 0.1µg/ml. 1µl vector DNA was ligated with 2µl insert DNA after adding 1µl 10x ligase buffer (0.5M TRIS-HCl, pH 7.8, 10mM $MgCl_2$, 100mM DTT, 50mM NaCl, 500 µM ATP) and 1µl DNA-ligase (1 Weiss-unit, Pharmacia, Uppsala) in a total volume of 10 µl. Ligations were carried out at 20°C for 1 hour. Control ligations with no insert DNA added were done in parallel.

E.coli K12 strain TB-1 was obtained from BRL (Messing strain kit). For the preparation of competent cells a single colony of TB-1 was grown in LB-medium [10g Bacto-tryptone, 5g Bacto-yeast extract, 10g NaCl per 1 liter, T. Maniatis et al., supra] overnight at 37°C in a rotary shaker. 500µl of that overnight culture were diluted in 100 ml LB-medium and grown 2 more hours at 37°C. The cells were collected by centrifugation for 10 min at 9000 rpm and 4°C. The pellet was suspended carefully in 20 ml of 50mM $CaCl_2$ and placed on ice for 30 min. The cells were centrifuged again and the pellet was dissolved in 10 ml of 50mM $CaCl_2$ containing 20% glycerol (v/v). The cells were shock frozen in 300µl aliquots and stored at -80°C for at least 3 months without notable loss of transformation efficiency.

For transformation 10µl of 0.5M $MgCl_2$ and 0.1M $CaCl_2$, 10µl of 30% polyethylenglycol, DNA and water were mixed to give a total volume of 100µl. Upon addition of 100µl thawed competent cells the samples were placed on ice for 20 min followed by a 10 min incubation at room temperature. After adding 1 ml of LB-medium the samples were incubated for 1 hour at 37°C waterbath. The cells were then centrifuged for 3 min at 12.000 rpm in a microfuge. The supernatant was withdrawn and the pellets were suspended in 100µl of LB-medium and plated on LB-agar plates containing 80µg/ml ampicillin. The plates were incubated overnight at 37°C.

After transformation of the subclone 1-4 ligation mixtures about 200 transformants per ligation were obtained. The control ligations gave no transformants. Single colonies were picked with a tooth pick, transferred to a tube containing LB-medium with 80µg/ml ampicillin and grown for 6 hours at 37°C with vigorous shaking. The cells were centrifuged per 10 min at 8000 rpm. The pellets were resuspended in 500µl of 50mM TRIS-HCl, pH 8.0 and 10mM EDTA. After addition of lysozyme to a final concentration of 1 mg/µl the samples were incubated at room temperature for 5 min. After adding 25µl of 10% SDS (sodium dodecyl sulphate) and 50µl 5M potassium acetate the samples were kept on ice for 15 min. The chromosomal DNA was then pelleted for 15 min at 12.000 rpm in a microfuge. The supernatants were transferred to new tubes and 1µl of RNase A (10mg/ml) was added followed by a 10 min incubation at 37°C. The DNA was extracted by adding an equal volume of phenol saturated with 1M TRIS-HCl, pH 8.0. The phases were separated by centrifugation at 12.000 rpm for 5 min. The supernatants were transferred to new tubes and an equal volume of chloroform was added. The phases were separated by centrifugation and the supernatants were transferred to new tubes. The DNA was then precipitated for 10 min at room temperature by adding 0.6 volumes of isopropanol and 0.1 volume of 5M lithium acetate. The samples were then centrifuged for 10 min at 12.000 rpm in the microfuge. The pellets were washed with 80% ethanol and dried in vacuo. The pellets were dissolved in 50µl water. 10µl were digested with the appropriate restriction enzyme (10 units) to release the inserts as described above in a total volume of 100µl. The DNA was then precipitated, washed with 80% ethanol and dried in vacuo. The pellets were dissolved in 10µl gel sample buffer and analyzed on a 6% polyacrylamide gel as described above with phage ϕX DNA as a size marker. The inserts of all four subclones had the expected size of 64, 55, 58 and 69 basepairs, respectively. The subclones were named pA1, pA2, pA3 and pA4.

D. <u>Construction of pA2-3</u>

0.2µg of pA2 and pA3 were digested with AatII/PstI in a total volume of 100µl using 5 units of each enzyme for 1 hour at 37°C. The samples were precipitated, washed with 80% ethanol and dried as described above. The pellets were dissolved in 10µl gel-loading buffer (see above) and electrophoresed on a 0.8% agarose gel containing 1xTBE and 1µg/ml ethidium bromide. The 500 bp fragment of pA2 and the 2 kb fragment of pA3 were isolated using the NA45 membrane and further purified as described above. The final pellets were dissolved in 5µl water. 2µl of each fragment were supplemented with 1µl 10x ligase buffer (0.5M TRIS-HCl, pH 7.8, 0.1M $MgCl_2$, 0.2M DTT, 10mM ATP) 1µl T4-DNA-ligase (1 Weiss-unit, Pharmacia, Uppsala) and 4µl water. Ligation was carried out for 1 hour at room temperature. The sample was then transformed into TB-1 as described above. Transformants were further analyzed by the "minilysate" procedure described above. The insert of the recombinant plasmids were released with EcoRI and HindIII and had the expected size of 114 basepairs as estimated by gel electrophoresis on an 8% polyacrylamide gel. The new recombinant plasmid, containing the blocks 2 and 3 of the env-gene fragment, was designated pA2-3.

E. Construction of pA1-2-3

0.2μg of pA1 and pA2-3 were digested with AatII/EcoRI in a total volume of 100 μl using 5 units of each enzyme for 1 hour at 37°C. The subsequent manipulation of the DNA was the same as for the construction of pA2-3 (see also figure 2). The insert of the new recombinant plasmid was released with HindIII since there is an additional HindIII site in the polylinker of pUC18. The insert had the expected size of about 200 basepairs. The new plasmid, now containing the gene blocks one, two and three was named pA1-2-3.

F. Construction and sequence analysis of pA-ENV-20

2μg of pA1-2-3 were digested with 20 units HindIII for 1 hour at 37°C in a total volume of 300μl. The DNA was precipitated and electrophoresed on a 1% agarose gel as described above. The 200 basepair fragment was isolated using the NA45 membrane and further purified as described above. The final pellet was suspended in 10μl water. The plasmid pA4 (0.5μg) was digested with 10 units HindIII in 100μl for 1 hour at 37°C. The ends were then dephosphorylated for 2 hours at 65°C by adding 10 units bacterial alkaline phosphatase, 40μl 1M TRIS-HCl, pH 8.0 and 160μl water. The sample was extracted three times with 400μl phenol saturated with 1M TRIS-HCl, pH 8.0. The phases were separated by centrifugation as described above. Following the third phenol extraction the DNA was precipitated for 10 min on dry ice after adding 40µl 5M lithium acetate and 800µl isopropanol. The sample was centrifuged for 10 min at 12.000 rpm in a microfuge. The supernatant was withdrawn and the pellet was washed three times with 80% ethanol and finally dried for 15 min in a speed-vac vacuum centrifuge. The pellet was dissolved in 5µl water. 2µl of pA-4 DNA which was digested with HindIII and dephosphorylated were ligated with 1µl HindIII-fragment of pA1-2-3 by adding 1µl T4-DNA-ligase (1 Weiss-unit, Pharmacia, Uppsala), 1µl 10x ligase buffer and 5µl water for 1 hour at room temperature. In parallel, a control ligation was done with no insert DNA from pA1-2-3. The ligation mixture was then transformed in TB-1. The sample containing the HindIII--insert gave about 500 recombinant colonies and the background with no HindIII insert was 20 colonies. 12 recombinant plasmids were isolated by the "minilysate"-procedure described above. After cutting with BamHI the insert with 246 base pairs was released from 10 out of 12 plasmids after polyacrylamide electrophoresis on 8% gels. Test digests with HindIII, PstI and EcoRI showed the presence of these restriction sites in the final plasmid pA-ENV-20.

E. coli TB-1 transformed with pA-ENV-20 was deposited at the Deutsche Sammlung von Mikroorganismen (DSM) in Göttingen on October 3, 1985, the accession no. being DSM 3516.

G. <u>Sequencing of the BamHI fragment of pA-ENV-20 in M13mp18</u>

M13mp18RFI DNA was purchased from Pharmacia (Uppsala) at a concentration of 0.1µg/ml. 10µl (1µg) were digested with 10 U BamHI in 100µl for 1 hour at 37°C. The DNA was dephosphorylated for 2 h at 65°C by adding 10 U bacterial alkaline phosphatase, 40µl 1M TRIS-HCl, pH 8.0 and 240µl water. The sample was phenol extracted and precipitated as described above. The pellet was dissolved in 20µl water. 2µg of pA-ENV-20 DNA were digested with BamHI (20 units) in a reaction volume of 200µl. The insert was isolated from an 1% agarose gel as described above. The final pellet was dissolved in 10µl water. 1µl M13mp18 RFI DNA cut with BamHI and dephosphorylated and 3µl pA-ENV-20 BamHI insert were ligated for 1 hour at room temperature after adding 5µl water, 1µl T4-DNA-ligase (1 Weiss-unit, Pharmacia, Uppsala) and 1µl 10xligase buffer. The sample was then transformed into the E.coli strain JM105 (BRL, Basel, strain kit) using the protocol described above. The 1 hour incubation after the transformation was omitted and the cells were plated directly on LB-plates in soft agar containing indicator-cells, IPTG (isopropyl ß-D-thiogalactopyranoside) and X-Gal (5-bromo-4-chloro-3-indolyl-ß-D--galactoside) as described in the M13 manual provided with the sequencing kit from BRL (Basel). A control ligation with no insert DNA was done in parallel. After overnight incubation at 37°C the control plate had 20 blue plaques. The plate with the insert had 23 blue plaques and in addition about 180 white plaques with insert. 3 white plaques were picked with with a toothpick and grown for 5 hours at 37°C with vigorous shaking in 3 ml LB-medium containing 10µl indicator cells (JM105, BRL, Basel, strain kit) of a fresh overnight culture. Single-stranded DNA templates were prepared from phage supernatants as described in the BRL-M13 manual. The templates were sequenced by the dideoxy method of Sanger using a 17-mer universal primer (Pharmacia, Uppsala). The reactions were carried out as described in the M13-manual of BRL including the gel electrophoresis on 0.4mm thick gels. After separating the glass plates the gel was fixed in 10% methanol, 10% acetic acid and 80% water for 15 min. The gel was then transferred to Whatman 3mm paper and dried on a gel dryer. The gel was exposed to Kodak-XAR film overnight at room temperature with no intensifying screen. All templates sequenced had the correct, predicted sequence of the synthetic gene env(80).

## Example 3

### Description of plasmids used for the expression of CAT, DHFR, ENV(80)-CAT, ENV(80)-DHFR and ENV(80)

### A. Principles

pDS5/RBSII,3A+5A (figure 3), pDS6/RBSII,3A+5A (figure 4) and pDS8/RBSII (figure 5) were chosen not only for the expression of derivatives of chloramphenicol acetyltransferase (CAT*) and dihydrofolate reductase (DHFR*), but also for the expression of the env(80)-polypeptide (ENV(80)) and the fusion proteins ENV(80)-CAT and ENV(80)-DHFR.
For efficient expression the above mentioned vectors contain the regulatable promoter/operator element $P_{N25*/O}$ [D. Stüber et al., The EMBO J., 3, pp. 3143-3148 (1984)] and the ribosomal binding sites RBSII or RBSII,3A+5A. These ribosomal binding sites have been derived via DNA-synthesis matching the ribosomal binding site under control of E.coli phage T5 promoter $P_{G25}$ [R. Gentz, Ph.D. thesis, University of Heidelberg, FRG (1984)]. Due to the high efficiency of these expression signals the above mentioned vectors can only be stably maintained in E.coli if the promoter/operator element is repressed via binding of the lac repressor to the operator entity of $P_{N25*/O}$. The lac repressor is encoded by the lacI gene. Since $P_{N25*/O}$ is efficiently repressed only if sufficient amounts of repressor molecules are present, the $lacI^q$ allel was used with a mutant promoter resulting in an increased transcription of the gene to provide enough repressor molecules. The $lacI^q$ allel is part of plasmid pDMI,1 (figure 6) which is compatible with the above mentioned plasmids and carries in addition the neo-gene conferring resistance to kanamycin as a selective marker. E.coli cells to be transformed with pDS5/RBSII,3A+5A, pDS6/RBSII,3A+5A, pDS8/RBSII or derivatives of these plasmids containing the ENV(80)-gene have therefore to harbour pDMI,1 to ensure stable maintenance of these vectors. Induction of expression in this system is easily achieved by addition of IPTG to the medium at the desired cell density.

B. Plasmid pDS5/RBSII,3A+5A

The part of pDS5/RBSII,3A+5A (figure 3) between the sites for restriction endonucleases XbaI and XhoI containing the region required for DNA replication and maintenance of the plasmid in the cell and the entire gene for ß-lactamase conferring resistance to ampicillin is pBR322 derived [F. Bolivar et al., Gene, 2, pp. 95-113 (1977); J.G. Sutcliffe, supra]. The remaining part of the plasmid carries the regulatable promoter/operator element $P_{N25*/0}$ (D. Stüber et al., supra) followed by ribosomal binding site RBSII,3A+5A which is part of an EcoRI/BamHI fragment, by sites for restriction endonucleases SalI, PstI and HindIII, by the promoter-free gene for chloramphenicol acetyltransferase [R. Marcoli et al., FEBS Letters, 110, pp. 11-14 (1980)] and by terminator T1 of the Escherichia coli rrnB operon [J. Brosius et al., J.Mol.Biol., 148, pp. 107-127 (1981)].

C. Plasmid pDS6/RBSII,3A+5A

Plasmid pDS6/RBSII,3A+5A (figure 4) corresponds to pDS5/RBSII,3A+5A (figure 3) but contains in addition in front of the cat-gene terminator $t_0$ of Escherichia coli phage Lambda [E. Schwarz et al., Nature, 272, pp. 410-414 (1978)].

D. Plasmid pDS8/RBSII

Plasmid pDS8/RBSII (figure 5) corresponds to pDS5/RBSII,3A+5A (figure 3) but contains between $P_{N25*/0}$ and the cat-gene the ribosomal binding site RBSII followed by the coding region for dihydrofolate reductase of mouse AT-3000 cell line [A.C.Y. Chang et al., Nature, 275, pp. 617-624 (1978); J.N Masters and G. Attardi, Gene, 21, pp. 59-63 (1983)] and by terminator $t_o$ of Escherichia coli phage Lambda [E. Schwarz et al., supra].

E. Plasmid pDMI,1

Plasmid pDMI,1 (figure 6) carries on a HindIII/SalI fragment the gene for neomycin phosphotransferase from transposon Tn5 [E. Beck et al., Gene, 19, pp. 327-336 (1982)] conferring resistance to kanamycin followed by the lacI gene [P.J. Farabaugh, Nature, 274, pp. 765-769 (1978)] with the promoter mutation $I^q$ [M.P. Calos, Nature, 274, pp. 762-765 (1978)] encoding the lac-repressor. Furthermore, pDMI,1 contains a region of plasmid pACYC184 [A.C.Y. Chang and S.N. Cohen, J.Bacteriol., 134, pp. 1141-1156 (1978)] carrying the information necessary for its replication and stable maintenance in E.coli.

## Example 4

### Construction of plasmids pENV(80), pENV(80)-CAT and pENV(80)-DHFR

A. Principles

Plasmids pENV(80)-CAT, pENV(80) and pENV(80)-DHFR were constructed by inserting the BamHI-fragment of pA-ENV-20 containing the ENV(80)-gene into the BamHI-site of plasmids pDS5/RBSII,3A+5A, pDS6/RBSII,3A+5A and pDS8/RBSII, respectively (figure 7).

B. Isolation of the BamHI-fragment of pA-ENV(80)-20 containing the ENV(80)-gene

1 pmole of the BamHI-fragment of pA-ENV(80)-20 containing the ENV(80)-gene was isolated as described above and resuspended in 10µl TE-buffer (10mM TRIS-HCl, pH 7.6 and 1mM EDTA).

C. Preparation of plasmids pDS5/RBSII,3A+5A, pDS6/RBSII,3A+5A and pDS8/RBSII

In three separate experiments 3 pmoles of plasmids pDS5/RBSII,3A+5A, pDS6/RBSII,3A+5A and pDS8/RBSII were digested to completion with restriction endonuclease BamHI. After inactivation of the enzyme by incubation for 7 minutes at 65°C and removal of the protein by extraction with phenol followed by treatment with ether, the DNA was precipitated with ethanol. The DNA was resuspended in 42µl buffer of pH 8 containing 50mM Tris/HCl and 1 unit calf intestinal phosphatase (CIP, Boehringer, Mannheim) and incubated for 1 hour at 37°C. After heat-inactivation of the enzyme (see above) and removal of the protein (see above) the DNA was precipitated with ethanol. After resuspension of the DNA, the linearized plasmid DNA was isolated via electrophoresis in 1% agarose gels, electro-transfer onto NA45 membranes, subsequent elution, ethanol precipitation and resuspension in 100µl TE-buffer (see above). Between 0.5 and 1.5 pmoles of linearized, dephosphorylated DNA of plasmids pDS5/RBSII,3A+5A, pDS6/RBSII,3A+5A and pDS8/RBSII were obtained.

D. Assembly of plasmids pENV(80)-CAT, pENV(80) and pENV(80)-DHFR

In three separate experiments 0.025 pmoles isolated DNA of plasmids pDS5/RBSII,3A+5A, pDS6/RBSII,3A+5A and pDS8/RBSII were incubated in a volume of 24µl in ligation buffer with 0.05 pmole of the isolated env(80)-gene and 1.8 units of T4-DNA ligase (Boehringer, Mannheim) for 6 hours at 20°C before the enzyme was inactivated by incubation for 7 minutes at 65°C.

E.coli M15 ($CaCl_2$ competent) harbouring pDMI,1 was transformed with the ligated DNA under appropriate conditions. After selection of transformants at 37°C on LB-plates containing 100µg/ml ampicillin and 25µg/ml kanamycin, cultures were grown in LB-medium containing 100µg/ml ampicillin and 25µg/ml kanamycin. DNA from these cultures was isolated using standard procedures and analyzed for the presence of the env(80)-gene and the orientation of this gene by restriction analysis using restriction endonucleases XhoI and HindIII. Plasmids pDS5/RBSII,3A+5A, pDS6/RBSII,3A+5A and pDS8/RBSII containing the env(80)-gene in the right orientation were designated pENV(80)-CAT, pENV(80) and pENV(80)-DHFR, respectively (figure 7).

## Example 5

## Recognition of proteins ENV(80)-CAT, ENV(80)-DHFR and ENV(80) by antibodies of a patient expressing AIDS

### A. Principles

To demonstrate that the env(80)-gene encodes an epitope which is specifically recognized by antibodies of a patient expressing AIDS, proteins CAT*, DHFR*, ENV(80)-CAT, ENV(80)-DHFR and ENV(80) were produced in E.coli, transferred to nylon-membranes and reacted with an appropriate serum.

### B. Production of proteins CAT*, DHFR*, ENV(80)-CAT, ENV(80)-DHFR and ENV(80) in E.coli

E.coli M15 cells containing plasmid pDMI,1 were transformed with either pDS5/RBSII,3A+5A, pDS6/RBSII,3A+5A, pDS8/RBSII, pENV(80)-CAT, pENV(80)-DHFR or pENV(80) and subsequently grown in LB-medium containing 100µg/ml ampicillin and 25µg/ml kanamycin. At an optical density at 600nm of about 0.7 the cultures were divided: one aliquot of the cultures was incubated without modification (uninduced samples) whereas to the second aliquot IPTG was added to a final concentration of 2 mM (induced samples). After additional 6 hours of incubation the cells were harvested by centrifugation.

C. Visualization of proteins produced in E.coli

Cells from a volume of 50µl of the cultures were resuspended in sample buffer containing 3% SDS, 3% β-Mercaptoethanol, 20% glycerol and 125 mM TRIS-HCl, pH 6.8. The samples were boiled for 5 minutes, chilled on ice, centrifuged at 12'000 xg for 30 seconds and electrophoresed in a SDS-containing polyacrylamide gel (12.5% acrylamide, ratio of acrylamide/bisacrylamide of 30/0.8) according to the procedure described by U.Laemmli [U.Laemmli, Nature 277, pp.680-682, (1970)]. After staining of the proteins with Coomassie brilliant Blue R-250 the unbound dye was removed from the gel. Comparing protein production under uninduced and induced conditions (see figure 9a), it is obvious, that the amount of protein which was synthesized under control of the regulatable promoter/operator element $P_{N25x10}$ is strongly increased in the presence of IPTG.

D. Reaction of antibodies with the produced proteins

Induced samples (see under B) were electrophoresed as described under C. The unstained gel was overlayed with a nylon-membrane (PAL, Basel). This sandwich was then covered with two sheets of filter paper and placed into a transfer chamber (Biorad, Glattbrugg) which was filled with transfer buffer (25mM TRIS-HCl, pH 8.0, 190mM glycine, 20% methanol), before the proteins were electrophoresed onto the membrane (12 hours at 25 V/cm, 4°C). After transfer the membrane was blocked for 2 hours at 37°C in PBS-buffer (125mM NaCl, 17.5mM $Na_2HPO_4$, 2.5mM $KH_2PO_4$) containing 20% foetal calf serum. This was followed by an 1 hour incubation at 20°C in PBS-buffer containing 0.3% Tween-80, 10% normal goat serum and 20% foetal calf serum, before the membrane was incubated for 45 minutes at 37°C and then for 16 hours at 4°C in the same buffer containing in addition a 1:200 dilution of patient serum 1330 provided by Dr.G.Hunsmann, Deutsches Primatenzentrum GmbH, Göttingen. The membrane was then washed at room temperature for 5, 10 and 15 minutes in PBS-buffer containing 0.3% Tween-80 with buffer change after each washing step. Afterwards, the membrane was incubated for 2 hours at room temperature in PBS-buffer containing 0.3% Tween-80, 10% foetal calf serum and a 1:200 dilution of goat antihuman IgG coupled with biotin (Amersham, Braunschweig). The three washes described above were repeated, before the membrane was incubated for 1 hour at room temperature in PBS-buffer containing 0.3% Tween-80, 10% foetal calf serum and a 1:300 dilution of streptavidin-biotin-peroxydase (Amersham, Braunschweig). The membrane was then washed two times for 5 minutes in PBS-buffer containing 0.3% Tween-80 followed by 5 washes for 5 minutes in PBS--buffer, before the membrane was left in 50ml PBS-buffer. Protein bands which reacted with antibodies were visualized by adding 10ml of 4-chloro-1-naphtol (3mg/ml in methanol) and 50µl of 30% hydrogenperoxide. The reaction was stopped by putting the membrane in water followed by drying in a gel dryer (Zabona, Basel). All washes and incubations were done on a shaker tablet (GFL, Burgwedel).

Figure 9b demonstrates that only those proteins which contain the 80 amino acids encoded by the ENV(80)-gene are recognized by the antibodies of the AIDS patient.

0219106

## Claims

1. A polypeptide comprising an amino acid sequence represented by the following formula:

MRGSEAQQHLLQLTVWGIKQLQARILAVERYLKDQQLLGIWGCSGKLICTTA
VPWNASWSNKLLEQIWNNMTWMEWDREINNYTGSVDLQPSLDSC (ENV(80)) I

or fragments thereof eliciting antibodies against AIDS virus and/or reacting with AIDS-antibodies or immunogenic and/or antigenic polypeptides related to any of the foregoing polypeptides by amino acid substitution(s).

2. A polypeptide according to claim 1 with the amino acid sequence of formula I.

3. A polypeptide according to claim 1 which is a fragment of the polypeptide of formula I.

4. A polypeptide according to claim 3 with an amino acid sequence represented by the following formula:

EAQQHLLQLTVWGIKQLQARILAVERYLKDQQLLGIWGCSGKLICTTA
VPWNASWSNKLLEQIWNNMTWMEWDREINNYT II

5. A polypeptide according to claim 1 related to the polypeptides of claims 2 to 4 by amino acid substitution(s).

6. A fusion polypeptide comprising a polypeptide according to any one of claims 1 to 5 fused to a prokaryotic or eukaryotic protein.

7. A fusion polypeptide according to claim 6 wherein the prokaryotic or eukaryotic protein is a carrier protein.

8. A fusion polypeptide according to claim 7 wherein the carrier protein is an easily purifiable protein.

9. A fusion polypeptide according to claim 8 wherein the carrier protein is E. coli chloramphenicol acetyltransferase (CAT).

10. A fusion polypeptide according to claim 9 which is ENV(80)-CAT.

11. A fusion polypeptide according to claim 8 wherein the carrier protein is dihydrofolate reductase (DHFR).

12. A fusion polypeptide according to claim 11 which is ENV(80)-DHFR.

13. A polypeptide according to any one of claims 1 to 12 with an additional methionine residue at the amino terminal.

14. A polypeptide according to any one of claims 1 to 13 bacterially produced.

15. A polypeptide according to any one of claims 1 to 13 produced by E. coli.

16. A synthetic gene coding for a polypeptide or fusion polypeptide as claimed in any one of claims 1 to 15.

17. An expression vector comprising a synthetic gene as claimed in claim 16 operatively linked to an expression DNA sequence.

18. An expression vector according to claim 17 which is a plasmid capable of replication in gram-negative and/or gram-positive bacteria.

19. An expression vector according to claim 18 which is capable of replication in an E. coli strain.

20. An expression vector according to claim 18 which is capable of replication in a B. subtilis strain.

21. An expression vector according to claim 18 or 19 which is a member of the plasmid pENV family.

22. An expression vector of claim 21 which is pENV(80).

23. An expression vector of claim 21 which is pENV(80)-CAT.

24. An expression vector of claim 21 which is pENV(80)-DHFR.

25. A bacterial transformant carrying an expression vector as claimed in any one of claims 18 to 24.

26. A transformant according to claim 25 which is an E. coli strain.

27. A transformant according to claim 26 which is an E. coli M15 strain.

28. A transformant according to claim 25 which is a B. subtilis strain.

29. A polypeptide according to any one of claims 1-15 as constituent of a vaccine.

30. A polypeptide according to any one of claims 1-15 as antigen.

31. A process for the manufacture of a polypeptide as claimed in any one of claims 1 to 15 which comprises transforming a bacterial host with an expression vector as claimed in any one of claims 17 to 24, culturing the transformant under appropriate conditions of growth so that said polypeptide is expressed and isolating said polypeptide.

32. A method for the determination of antibodies against AIDS virus which comprises using a polypeptide as claimed in any one of claims 1 to 15 in a manner known in the art.

33. A method according to claim 32 which comprises the use of the Western Blotting Analysis.

34. A method according to claim 32 which comprises the use of an ELISA-technique, wherein the polypeptide as claimed in any one of claims 1 to 15 is coated on a solid phase and contacted with the sample and after washing contacted with an enzyme-labeled non-human IgG.

35. A method according to claim 32, wherein the Double--Antigen-Method is used.

36. A method for the determination of AIDS virus, wherein antibodies against a protein according to any one of claims 1 to 15 are used.

37. A method according to claim 36, wherein the antigen in the sample and a polypeptide as claimed in any one of claims 1 to 15 in labeled form compete with an antibody against a polypeptide as claimed in any one of claims 1 to 15.

38. A method according to claim 36, wherein a sandwich method is performed using two antibodies against a polypeptide as claimed in any one of claims 1 to 15.

39. A method according to claim 38, wherein one antibody is on a solid phase and the other antibody is labeled.

40. A method according to claim 38, wherein the two antibodies are produced in different animal species.

41. A method according to claim 38, wherein two different monoclonal antibodies are used.

42. Vaccines containing a polypeptide as claimed in any one of claims 1 to 15 and a physiologically acceptable carrier.

43. Antibodies raised against a polypeptid as claimed in any one of claims 1 to 15.

44. The antibodies of claim 43 which are monoclonal antibodies.

45. The use of a polypeptid as claimed in any one of claims 1 to 15 for the preparation of a protective immunization vaccine.

46. The use of a polypeptid as claimed in any one of claims 1 to 15 for the preparation of antibodies against AIDS virus.

47. The use of a polypeptid as claimed in any one of claims 1 to 15 for testing human blood for the presence of AIDS virus.

48. A test kit for the determination of antibodies against AIDS virus comprising in a container a polypetide according to any one of claims 1 to 15.

49. A test kit for the determination of AIDS virus comprising in a container antibodies against AIDS virus elicited by a polypeptide according to any one of claims 1 to 15.

Figure 1

```
BamHI ---------- 1 ---------- EcoRI ---------- 2 ---------- PstI
GATCCGAAGCTCAACAGCATCTGCTGCAACTCACTGTTTGGGGTATCAAACAGCTCCAGGCTCGAATTCTGGCTGTTGAACGTTACCTGAAAGATCAACAGCTCCTGGGTATCTGGGGCTGCAGT
    GCTTCGAGTTGTCGTAGACGACGTTGAGTGACAAACCCCATAGTTTGTCGAGGTCCGAGCTTAAGACCGACAACTTGCAATGGACTTTCTAGTTGTCGAGGACCCATAGACCCCGACGTCA

---------- 3 ---------- HindIII ---------- 4 ---------- BamHI
GGTAAACTCATCTGCACTACTGCTGTTCCTTGGAATGCTTCTTGGTCTAATAAGCTTCTGGAACAGATCTGGAATAACATGACTTGGATGGAGTGGGACCGTGAAATCAACAATTACACTG
CCATTTGAGTAGACGTGATGACGACAAGGAACCTTACGAAGAACCAGATTATTCGAAGACCTTGTCTAGACCTTATTGTACTGAACCTACCTCACCCTGGCACTTTAGTTGTTAATGTGACCTAG
```

Fig. 2

0219106

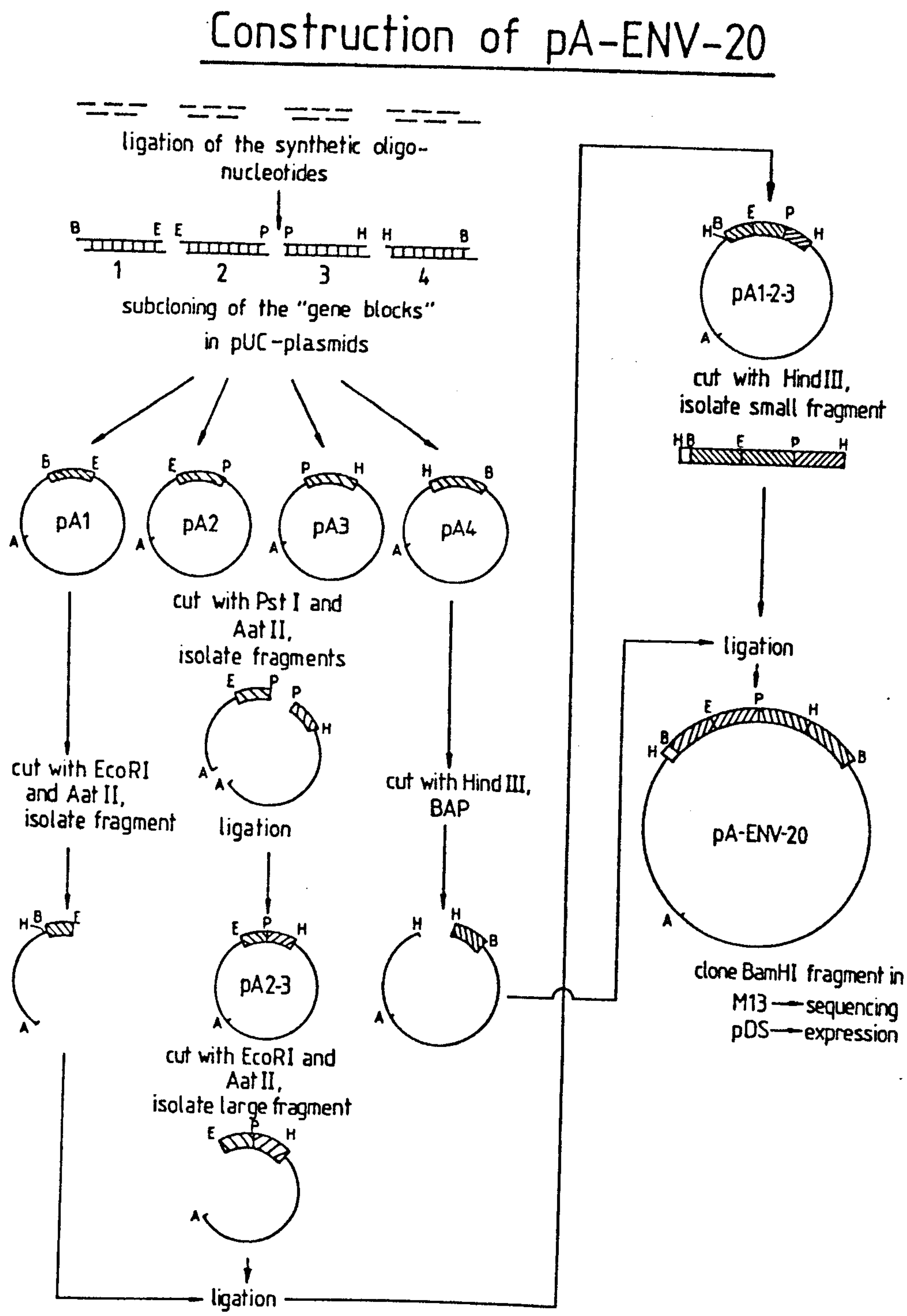

Fig. 3a



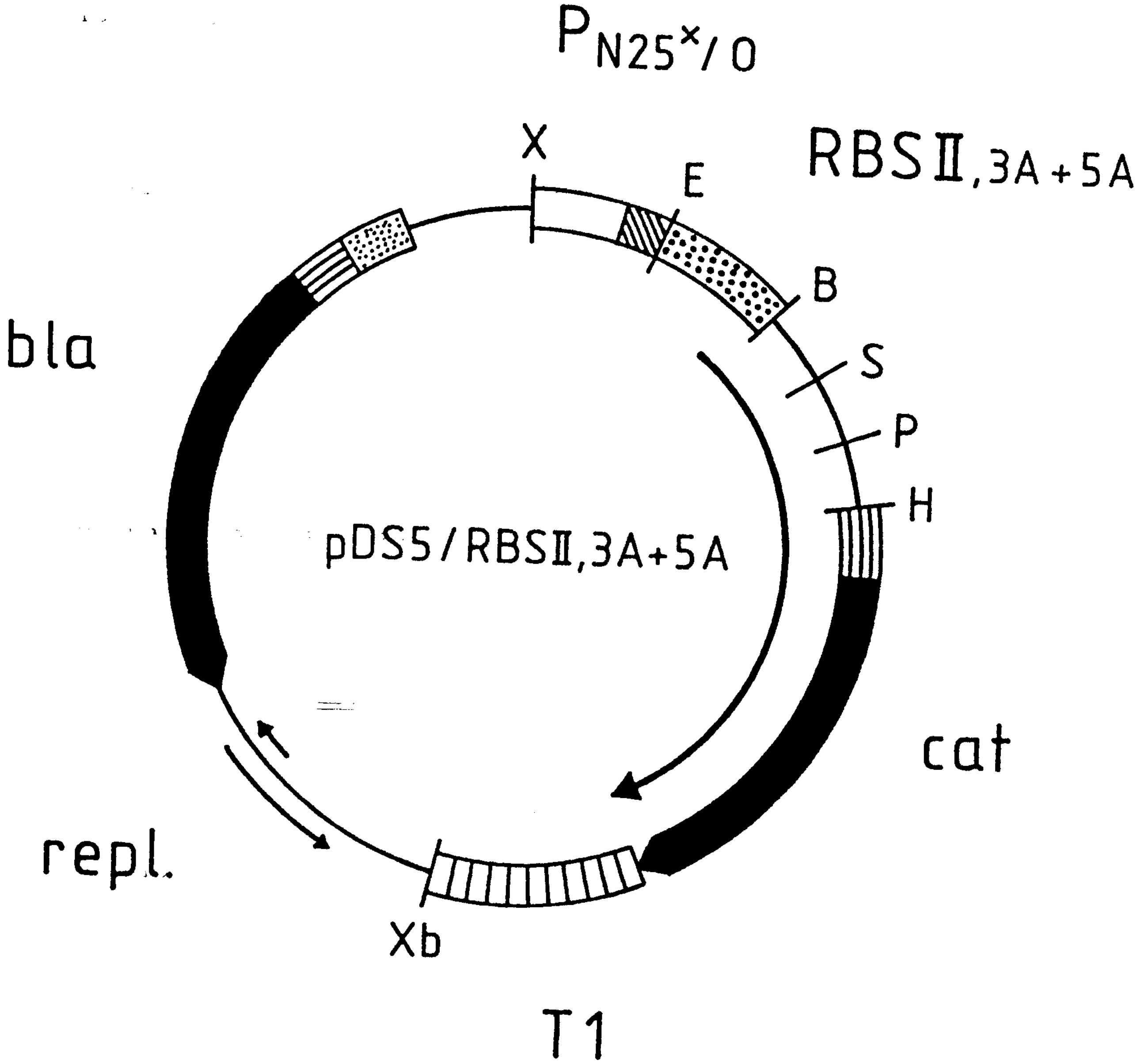

Fig. 3b



```
             10         20         30         40         50
   0 CCTCGAGGCT GGCATCCCTA ACATATCCGA ATGGTTACTT AAACAACGGA
  50 GGACTAGCGT ATCCCTTCGC ATAGGGTTTG AGTTAGATAA AGTATATGCT
 100 GAACTTTCTT CTTTGCTCAA AGAATCATAA AAAATTTATT TGCTTTCAGG
 150 AAAATTTTTC TGTATAATAG ATTCAAATTG TGAGCGGATA ACAATTTGAA
 200 TTCATTAAAG AGGAGAAATT AACTATGAGG GGATCCGTCG ACCTGCAGCC
 250 AAGCTTCGCG AGATTTTCAG GAGCTAAGGA AGCTAAAATG GAGAAAAAAA
 300 TCACTGGATA TACCACCGTT GATATATCCC AATGGCATCG TAAAGAACAT
 350 TTTGAGGCAT TTCAGTCAGT TGCTCAATGT ACCTATAACC AGACCGTTCA
 400 GCTGGATATT ACGGCCTTTT TAAAGACCGT AAAGAAAAAT AAGCACAAGT
 450 TTTATCCGGC CTTTATTCAC ATTCTTGCCC GCCTGATGAA TGCTCATCCG
 500 GAATTTCGTA TGGCAATGAA AGACGGTGAG CTGGTGATAT GGGATAGTGT
 550 TCACCCTTGT TACACCGTTT TCCATGAGCA AACTGAAACG TTTTCATCGC
 600 TCTGGAGTGA ATACCACGAC GATTTCCGGC AGTTTCTACA CATATATTCG
 650 CAAGATGTGG CGTGTTACGG TGAAAACCTG GCCTATTTCC CTAAAGGGTT
 700 TATTGAGAAT ATGTTTTTCG TCTCAGCCAA TCCCTGGGTG AGTTTCACCA
 750 GTTTTGATTT AAACGTGGCC AATATGGACA ACTTCTTCGC CCCCGTTTTC
 800 ACCATGGGCA AATATTATAC GCAAGGCGAC AAGGTGCTGA TGCCGCTGGC
 850 GATTCAGGTT CATCATGCCG TCTGTGATGG CTTCCATGTC GGCAGAATGC
 900 TTAATGAATT ACAACAGTAC TGCGATGAGT GGCAGGGCGG GGCGTAATTT
 950 TTTTAAGGCA GTTATTGGTG CCCTTAAACG CCTGGGGTAA TGACTCTCTA
1000 GCTTGAGGCA TCAAATAAAA CGAAAGGCTC AGTCGAAAGA CTGGGCCTTT
1050 CGTTTTATCT GTTGTTTGTC GGTGAACGCT CTCCTGAGTA GGACAAATCC
1100 GCCGCTCTAG AGC
                 2068

        pBR322                                   A
                                              4358
```

Fig. 4a



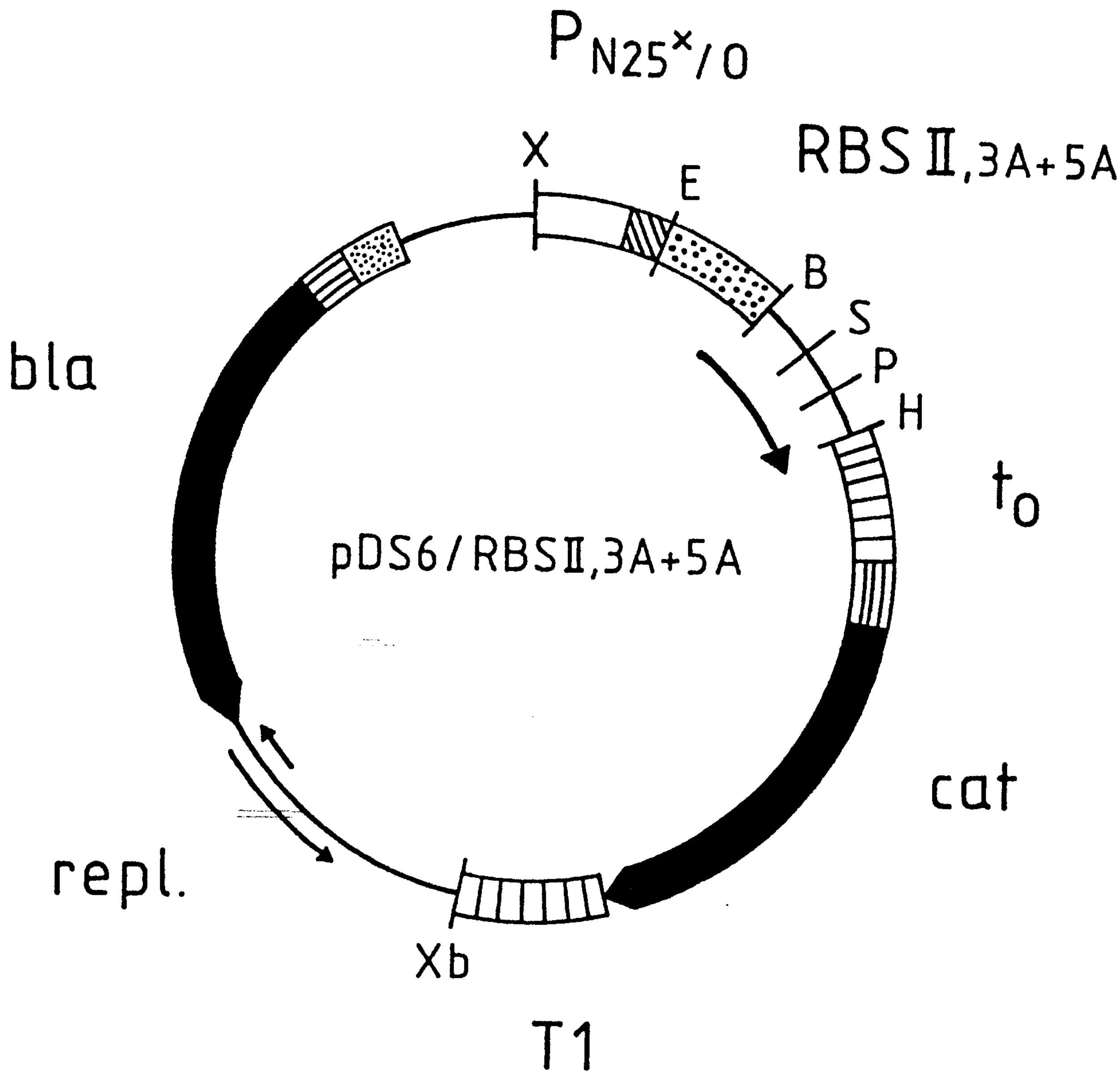
$P_{N25^x/0}$
RBSII,3A+5A
X
E
B
S
P
H
$t_0$
bla
pDS6/RBSII,3A+5A
cat
repl.
Xb
T1

Fig. 4b



| | 10 | 20 | 30 | 40 | 50 |
|---|---|---|---|---|---|
| 0 | CCTCGAGGCT | GGCATCCCTA | ACATATCCGA | ATGGTTACTT | AAACAACGGA |
| 50 | GGACTAGCGT | ATCCCTTCGC | ATAGGGTTTG | AGTTAGATAA | AGTATATGCT |
| 100 | GAACTTTCTT | CTTTGCTCAA | AGAATCATAA | AAAATTTATT | TGCTTTCAGG |
| 150 | AAAATTTTTC | TGTATAATAG | ATTCAAATTG | TGAGCGGATA | ACAATTTGAA |
| 200 | TTCATTAAAG | AGGAGAAATT | AACTATGAGG | GGATCCGTCG | ACCTGCAGCC |
| 250 | AAGCTTGGAC | TCCTGTTGAT | AGATCCAGTA | ATGACCTCAG | AACTCCATCT |
| 300 | GGATTTGTTC | AGAACGCTCG | GTTGCCGCCG | GGCGTTTTTT | ATTGGTGAGA |
| 350 | ATCCAAGCTA | GCTTGGCGAG | ATTTTCAGGA | GCTAAGGAAG | CTAAAATGGA |
| 400 | GAAAAAAATC | ACTGGATATA | CCACCGTTGA | TATATCCCAA | TGGCATCGTA |
| 450 | AAGAACATTT | TGAGGCATTT | CAGTCAGTTG | CTCAATGTAC | CTATAACCAG |
| 500 | ACCGTTCAGC | TGGATATTAC | GGCCTTTTTA | AAGACCGTAA | AGAAAAATAA |
| 550 | GCACAAGTTT | TATCCGGCCT | TTATTCACAT | TCTTGCCCGC | CTGATGAATG |
| 600 | CTCATCCGGA | ATTTCGTATG | GCAATGAAAG | ACGGTGAGCT | GGTGATATGG |
| 650 | GATAGTGTTC | ACCCTTGTTA | CACCGTTTTC | CATGAGCAAA | CTGAAACGTT |
| 700 | TTCATCGCTC | TGGAGTGAAT | ACCACGACGA | TTTCCGGCAG | TTTCTACACA |
| 750 | TATATTCGCA | AGATGTGGCG | TGTTACGGTG | AAAACCTGGC | CTATTTCCCT |
| 800 | AAAGGGTTTA | TTGAGAATAT | GTTTTTCGTC | TCAGCCAATC | CCTGGGTGAG |
| 850 | TTTCACCAGT | TTTGATTTAA | ACGTGGCCAA | TATGGACAAC | TTCTTCGCCC |
| 900 | CCGTTTTCAC | CATGGGCAAA | TATTATACGC | AAGGCGACAA | GGTGCTGATG |
| 950 | CCGCTGGCGA | TTCAGGTTCA | TCATGCCGTC | TGTGATGGCT | TCCATGTCGG |
| 1000 | CAGAATGCTT | AATGAATTAC | AACAGTACTG | CGATGAGTGG | CAGGGCGGGG |
| 1050 | CGTAATTTTT | TTAAGGCAGT | TATTGGTGCC | CTTAAACGCC | TGGGGTAATG |
| 1100 | ACTCTCTAGC | TTGAGGCATC | AAATAAAACG | AAAGGCTCAG | TCGAAAGACT |
| 1150 | GGGCCTTTCG | TTTTATCTGT | TGTTTGTCGG | TGAACGCTCT | CCTGAGTAGG |
| 1200 | ACAAATCCGC | CGCTCTAGAG | C | | |

2068

pBR322 A

4358

7/15

Fig. 5a



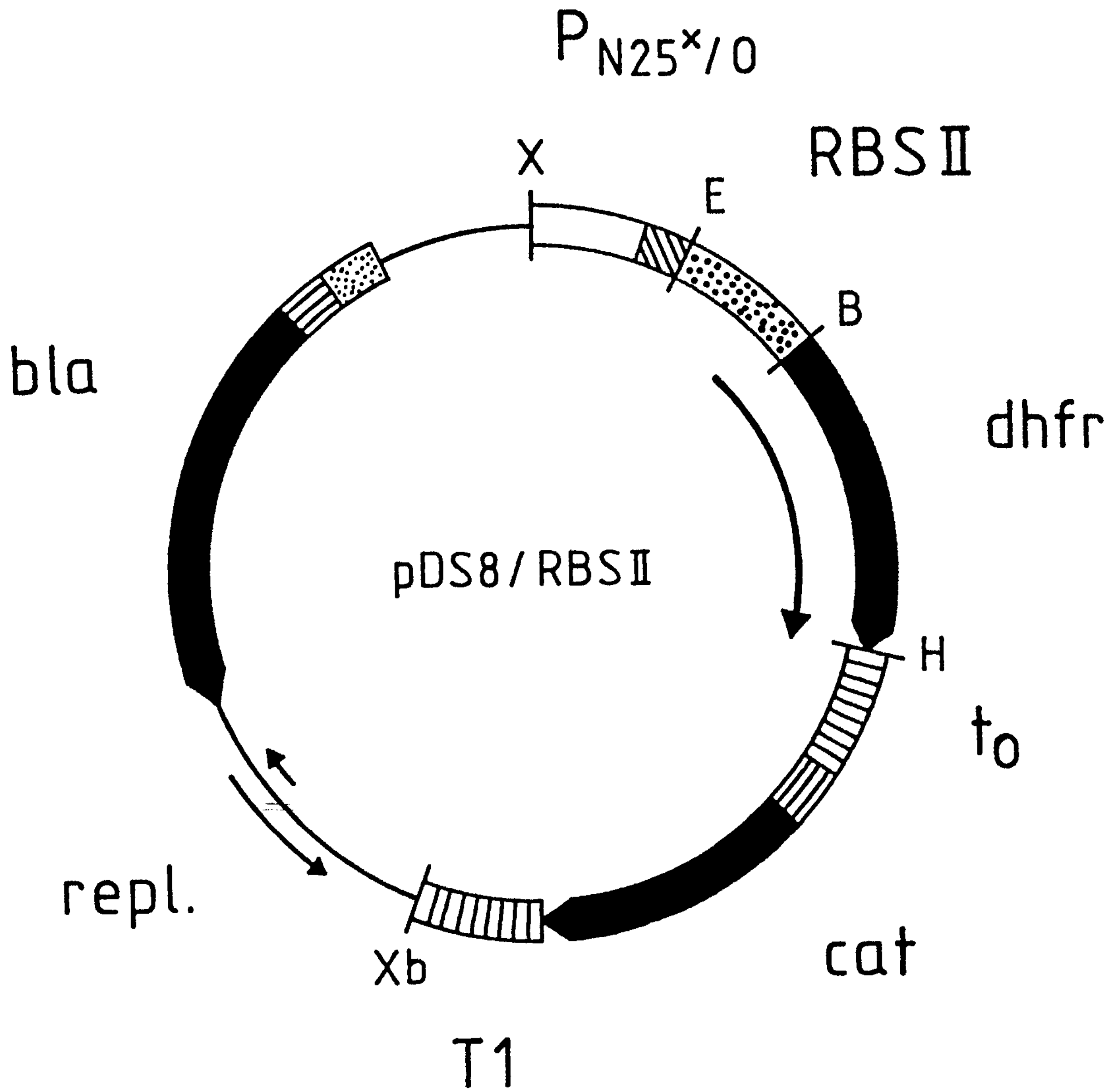

8/15

Fig. 5b

0219106

```
          10          20          30          40          50
   0 CCTCGAGGCT GGCATCCCTA ACATATCCGA ATGGTTACTT AAACAACGGA
  50 GGACTAGCGT ATCCCTTCGC ATAGGGTTTG AGTTAGATAA AGTATATGCT
 100 GAACTTTCTT CTTTGCTCAA AGAATCATAA AAAATTTATT TGCTTTCAGG
 150 AAAATTTTTC TGTATAATAG ATTCAAATTG TGAGCGGATA ACAATTTGAA
 200 TTCATTAAAG AGGAGAAATT AACTATGAGA GGATCCGGCA TCATGGTTCG
 250 ACCATTGAAC TGCATCGTCG CCGTGTCCCA AAATATGGGG ATTGGCAAGA
 300 ACGGAGACCT ACCCTGGCCT CCGCTCAGGA ACGAGTTCAA GTACTTCCAA
 350 AGAATGACCA CAACCTCTTC AGTGGAAGGT AAACAGAATC TGGTGATTAT
 400 GGGTAGGAAA ACCTGGTTCT CCATTCCTGA GAAGAATCGA CCTTTAAAGG
 450 ACAGAATTAA TATAGTTCTC AGTAGAGAAC TCAAAGAACC ACCACGAGGA
 500 GCTCATTTTC TTGCCAAAAG TTTGGATGAT GCCTTAAGAC TTATTGAACA
 550 ACCGGAATTG GCAAGTAAAG TAGACATGGT TTGGATAGTC GGAGGCAGTT
 600 CTGTTTACCA GGAAGCCATG AATCAACCAG GCCACCTTAG ACTCTTTGTG
 650 ACAAGGATCA TGCAGGAATT TGAAAGTGAC ACGTTTTTCC CAGAAATTGA
 700 TTTGGGGAAA TATAAACTTC TCCCAGAATA CCCAGGCGTC CTCTCTGAGG
 750 TCCAGGAGGA AAAAGGCATC AAGTATAAGT TTGAAGTCTA CGAGAAGAAA
 800 GACTAACAGG AAGATGCTTT CAAGTTCTCT GCTCCCCTCC TAAAGCTATG
 850 CATTTTTATA AGACCATGGG ACTTTTGCTG GCTTTAGATC CGGCC

 895 AAGCTTGGAC TCCTGTTGAT AGATCCAGTA ATGACCTCAG AACTCCATCT
 945 GGATTTGTTC AGAACGCTCG GTTGCCGCCG GGCGTTTTTT ATTGGTGAGA
 995 ATCCAAGCTA GCTTGGCGAG ATTTTCAGGA GCTAAGGAAG CTAAAATGGA
1045 GAAAAAAATC ACTGGATATA CCACCGTTGA TATATCCCAA TGGCATCGTA
1095 AAGAACATTT TGAGGCATTT CAGTCAGTTG CTCAATGTAC CTATAACCAG
1145 ACCGTTCAGC TGGATATTAC GGCCTTTTTA AAGACCGTAA AGAAAAATAA
1195 GCACAAGTTT TATCCGGCCT TTATTCACAT TCTTGCCCGC CTGATGAATG
1245 CTCATCCGGA ATTTCGTATG GCAATGAAAG ACGGTGAGCT GGTGATATGG
1295 GATAGTGTTC ACCCTTGTTA CACCGTTTTC CATGAGCAAA CTGAAACGTT
1345 TTCATCGCTC TGGAGTGAAT ACCACGACGA TTTCCGGCAG TTTCTACACA
1395 TATATTCGCA AGATGTGGCG TGTTACGGTG AAAACCTGGC CTATTTCCCT
1445 AAAGGGTTTA TTGAGAATAT GTTTTTCGTC TCAGCCAATC CCTGGGTGAG
1495 TTTCACCAGT TTTGATTTAA ACGTGGCCAA TATGGACAAC TTCTTCGCCC
1545 CCGTTTTCAC CATGGGCAAA TATTATACGC AAGGCGACAA GGTGCTGATG
1595 CCGCTGGCGA TTCAGGTTCA TCATGCCGTC TGTGATGGCT TCCATGTCGG
1645 CAGAATGCTT AATGAATTAC AACAGTACTG CGATGAGTGG CAGGGCGGGG
1695 CGTAATTTTT TTAAGGCAGT TATTGGTGCC CTTAAACGCC TGGGGTAATG
1745 ACTCTCTAGC TTGAGGCATC AAATAAAACG AAAGGCTCAG TCGAAAGACT
1795 GGGCCTTTCG TTTTATCTGT TGTTTGTCGG TGAACGCTCT CCTGAGTAGG
1845 ACAAATCCGC CGCTCTAGAG C ————————————————————
                           |
                          2068

——————pBR322———————————————————————————— A
                                         |
                                        4358
```

Fig. 6a



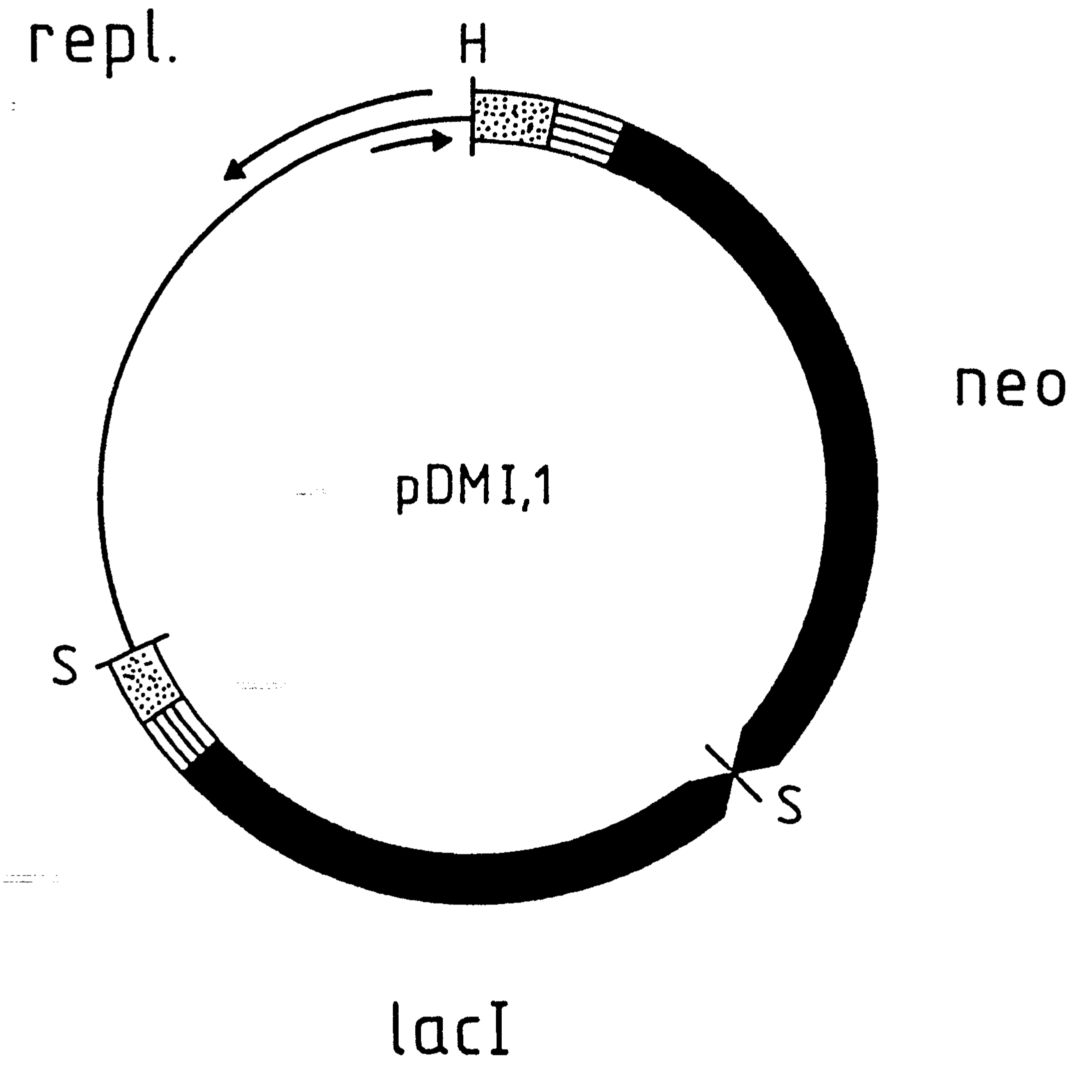

Fig. 6b

0219106

10 20 30 40 50

0 AAGCTTCACG CTGCCGCAAG CACTCAGGGC GCAAGGGCTG CTAA[illegible]

50 CGGAACACGT AGAAAGCCAG TCCGCAGAAA CGGTGCTGAC CCCGGATGAA

100 TGTCAGCTAC TGGGCTATCT GGACAAGGGA AAACGCAAGC GCAAAGAGAA

150 AGCAGGTAGC TTGCAGTGGG CTTACATGGC GATAGCTAGA CTGGGCGGTT

200 TTATGGACAG CAAGCGAACC GGAATTGCCA GCTGGGGCGC CCTCTGGTAA

250 GGTTGGGAAG CCCTGCAAAG TAAACTGGAT GGCTTTCTTG CCGCCAAGGA

300 TCTGATGGCG CAGGGGATCA AGATCTGATC AAGAGACAGG ATGAGGATCG

350 TTTCGCATGA TTGAACAAGA TGGATTGCAC GCAGGTTCTC CGGCCGCTTG

400 GGTGGAGAGG CTATTCGGCT ATGACTGGGC ACAACAGACA ATCGGCTGCT

450 CTGATGCCGC CGTGTTCCGG CTGTCAGCGC AGGGGCGCCC GGTTCTTTTT

500 GTCAAGACCG ACCTGTCCGG TGCCCTGAAT GAACTGCAGG ACGAGGCAGC

550 GCGGCTATCG TGGCTGGCCA CGACGGGCGT TCCTTGCGCA GCTGTGCTCG

600 ACGTTGTCAC TGAAGCGGGA AGGGACTGGC TGCTATTGGG CGAAGTGCCG

650 GGGCAGGATC TCCTGTCATC TCACCTTGCT CCTGCCGAGA AAGTATCCAT

700 CATGGCTGAT GCAATGCGGC GGCTGCATAC GCTTGATCCG GCTACCTGCC

750 CATTCGACCA CCAAGCGAAA CATCGCATCG AGCGAGCACG TACTCGGATG

800 GAAGCCGGTC TTGTCGATCA GGATGATCTG GACGAAGAGC ATCAGGGGCT

850 CGCGCCAGCC GAACTGTTCG CCAGGCTCAA GGCGCGCATG CCCGACGGCG

900 AGGATCTCGT CGTGACCCAT GGCGATGCCT GCTTGCCGAA TATCATGGTG

950 GAAAATGGCC GCTTTTCTGG ATTCATCGAC TGTGGCCGGC TGGGTGTGGC

1000 GGACCGCTAT CAGGACATAG CGTTGGCTAC CCGTGATATT GCTGAAGAGC

1050 TTGGCGGCGA ATGGGCTGAC CGCTTCCTCG TGCTTTACGG TATCGCCGCT

1100 CCCGATTCGC AGCGCATCGC CTTCTATCGC CTTCTTGACG AGTTCTTCTG

1150 AGCGGGACTC TGGGGTTCGA AATGACCGAC CAAGCGACGC CCAACCTGCC

1200 ATCACGAGAT TTCGATTCCA CCGCCGCCTT CTATGAAAGG TTGGGCTTCG

1250 GAATCGTTTT CCGGGACGCC GGCTGGATGA TCCTCCAGCG CGGGGATCTC

1300 ATGCTGGAGT TCTTCGCCCA CCCCGGGCTC GATCCCCTCG CGAGTTGGTT

1350 CAGCTGCTGC CTGAGGCTGG ACGACCTCGC GGAGTTCTAC CGGCAGTGCA

1400 AATCCGTCGG CATCCAGGAA ACCAGCAGCG GCTATCCGCG CATCCATGCC

1450 CCCGAACTGC AGGAGTGGGG AGGCACGATG GCCGCTTTGG TCGACAATTC

1500 GCGCTAACTT ACATTAATTG CGTTGCGCTC ACTGCCCGCT TTCCAGTCGG

1550 GAAACCTGTC GTGCCAGCTG CATTAATGAA TCGGCCAACG CGCGGGGAGA

1600 GGCGGTTTGC GTATTGGGCG CCAGGGTGGT TTTTCTTTTC ACCAGTGAGA

1650 CGGGCAACAG CTGATTGCCC TTCACCGCCT GGCCCTGAGA GAGTTGCAGC

1700 AAGCGGTCCA CGCTGGTTTG CCCCAGCAGG CGAAAATCCT GTTTGATGGT

1750 GGTTAACGGC GGGATATAAC ATGAGCTGTC TTCGGTATCG TCGTATCCCA

1800 CTACCGAGAT ATCCGCACCA ACGCGCAGCC CGGACTCGGT AATGGCGCGC

1850 ATTGCGCCCA GCGCCATCTG ATCGTTGGCA ACCAGCATCG CAGTGGGAAC

11/15

Fig. 6c

0219106

10 20 30 40 50

1900 GATGCCCTCA TTCAGCATTT GCATGGTTTG TTGAAAACCG GACATGGCAC
1950 TCCAGTCGCC TTCCCGTTCC GCTATCGGCT GAATTTGATT GCGAGTGAGA
2000 TATTTATGCC AGCCAGCCAG ACGCAGACGC GCCGAGACAG AACTTAATGG
2050 GCCCGCTAAC AGCGCGATTT GCTGGTGACC CAATGCGACC AGATGCTCCA
2100 CGCCCAGTCG CGTACCGTCT TCATGGGAGA AAATAATACT GTTGATGGGT
2150 GTCTGGTCAG AGACATCAAG AAATAACGCC GGAACATTAG TGCAGGCAGC
2200 TTCCACAGCA ATGGCATCCT GGTCATCCAG CGGATAGTTA ATGATCAGCC
2250 CACTGACGCG TTGCGCGAGA AGATTGTGCA CCGCCGCTTT ACAGGCTTCG
2300 ACGCCGCTTC GTTCTACCAT CGACACCACC ACGCTGGCAC CCAGTTGATC
2350 GGCGCGAGAT TTAATCGCCG CGACAATTTG CGACGGCGCG TGCAGGGCCA
2400 GACTGGAGGT GGCAACGCCA ATCAGCAACG ACTGTTTGCC CGCCAGTTGT
2450 TGTGCCACGC GGTTGGGAAT GTAATTCAGC TCCGCCATCG CCGCTTCCAC
2500 TTTTTCCCGC GTTTTCGCAG AAACGTGGCT GGCCTGGTTC ACCACGCGGG
2550 AAACGGTCTG ATAAGAGACA CCGGCATACT CTGCGACATC GTATAACGTT
2600 ACTGGTTTCA CATTCACCAC CCTGAATTGA CTCTCTTCCG GGCGCTATCA
2650 TGCCATACCG CGAAAGGTTT TGCACCATTC GATGGTGTCA ACGTAAATGC
2700 ATGCCGCTTC GCCTTCGCGC GCGAATTGTC GACCCTGTCC CTCCTGTTCA
2750 GCTACTGACG GGGTGGTGCG TAACGGCAAA AGCACCGCCG GACATCAGCG
2800 CTAGCGGAGT GTATACTGGC TTACTATGTT GGCACTGATG AGGGTGTCAG
2850 TGAAGTGCTT CATGTGGCAG GAGAAAAAAG GCTGCACCGG TGCGTCAGCA
2900 GAATATGTGA TACAGGATAT ATTCCGCTTC CTCGCTCACT GACTCGCTAC
2950 GCTCGGTCGT TCGACTGCGG CGAGCGGAAA TGGCTTACGA ACGGGGCGGA
3000 GATTTCCTGG AAGATGCCAG GAAGATACTT AACAGGGAAG TGAGAGGGCC
3050 GCGGCAAAGC CGTTTTTCCA TAGGCTCCGC CCCCCTGACA AGCATCACGA
3100 AATCTGACGC TCAAATCAGT GGTGGCGAAA CCCGACAGGA CTATAAAGAT
3150 ACCAGGCGTT TCCCCTGGCG GCTCCCTCGT GCGCTCTCCT GTTCCTGCCT
3200 TTCGGTTTAC CGGTGTCATT CCGCTGTTAT GGCCGCGTTT GTCTCATTCC
3250 ACGCCTGACA CTCAGTTCCG GGTAGGCAGT TCGCTCCAAG CTGGACTGTA
3300 TGCACGAACC CCCCGTTCAG TCCGACCGCT GCGCCTTATC CGGTAACTAT
3350 CGTCTTGAGT CCAACCCGGA AAGACATGCA AAAGCACCAC TGGCAGCAGC
3400 CACTGGTAAT TGATTTAGAG GAGTTAGTCT TGAAGTCATG CGCCGGTTAA
3450 GGCTAAACTG AAAGGACAAG TTTTGGTGAC TGCGCTCCTC CAAGCCAGTT
3500 ACCTCGGTTC AAAGAGTTGG TAGCTCAGAG AACCTTCGAA AAACCGCCCT
3550 GCAAGGCGGT TTTTTCGTTT TCAGAGCAAG AGATTACGCG CAGACCAAAA
3600 CGATCTCAAG AAGATCATCT TATTAATCAG ATAAAATATT TCTAGATTTC
3650 AGTGCAATTT ATCTCTTCAA ATGTAGCACC TGAAGTCAGC CCCATACGAT
3700 ATAAGTTGTT AATTCTCATG TTTGACAGCT TATCATCGAT

12/15

Fig. 7

0219106

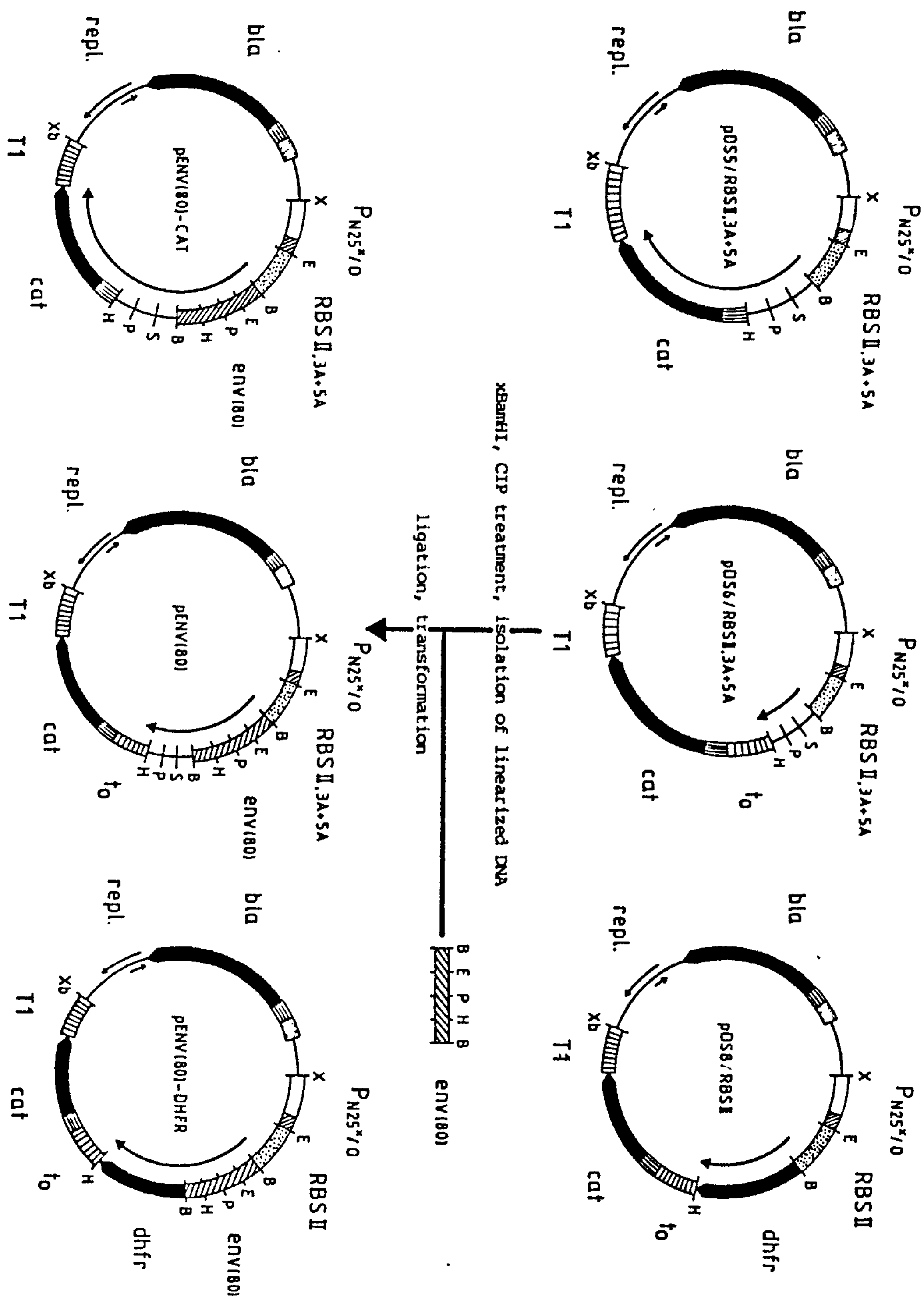

Fig. 8

| | 10 20 30 40 50 60 70 80 90 100 | |
|---|---|---|
| ENV(80) | MRGSEAQQHLLQLTVWGIKQLQARILAVERYLKDQQLLGIWGCSGKLICTTAVPWNASWSNKLLEQIWNNMTWMEWDREINNYTGSVDLQPSLDSC | |
| ENV(80)-CAT | MRGSEAQQHLLQLTVWGIKQLQARILAVERYLKDQQLLGIWGCSGKLICTTAVPWNASWSNKLLEQIWNNMTWMEWDREINNYTGSVDLQPSLARFSGAK | [ + 222 aa ] |
| ENV(80)-DHFR | MRGSEAQQHLLQLTVWGIKQLQARILAVERYLKDQQLLGIWGCSGKLICTTAVPWNASWSNKLLEQIWNNMTWMEWDREINNYTGSGIMVRPLNCIVAVS | [ + 175 aa ] |

Fig. 9

0219106

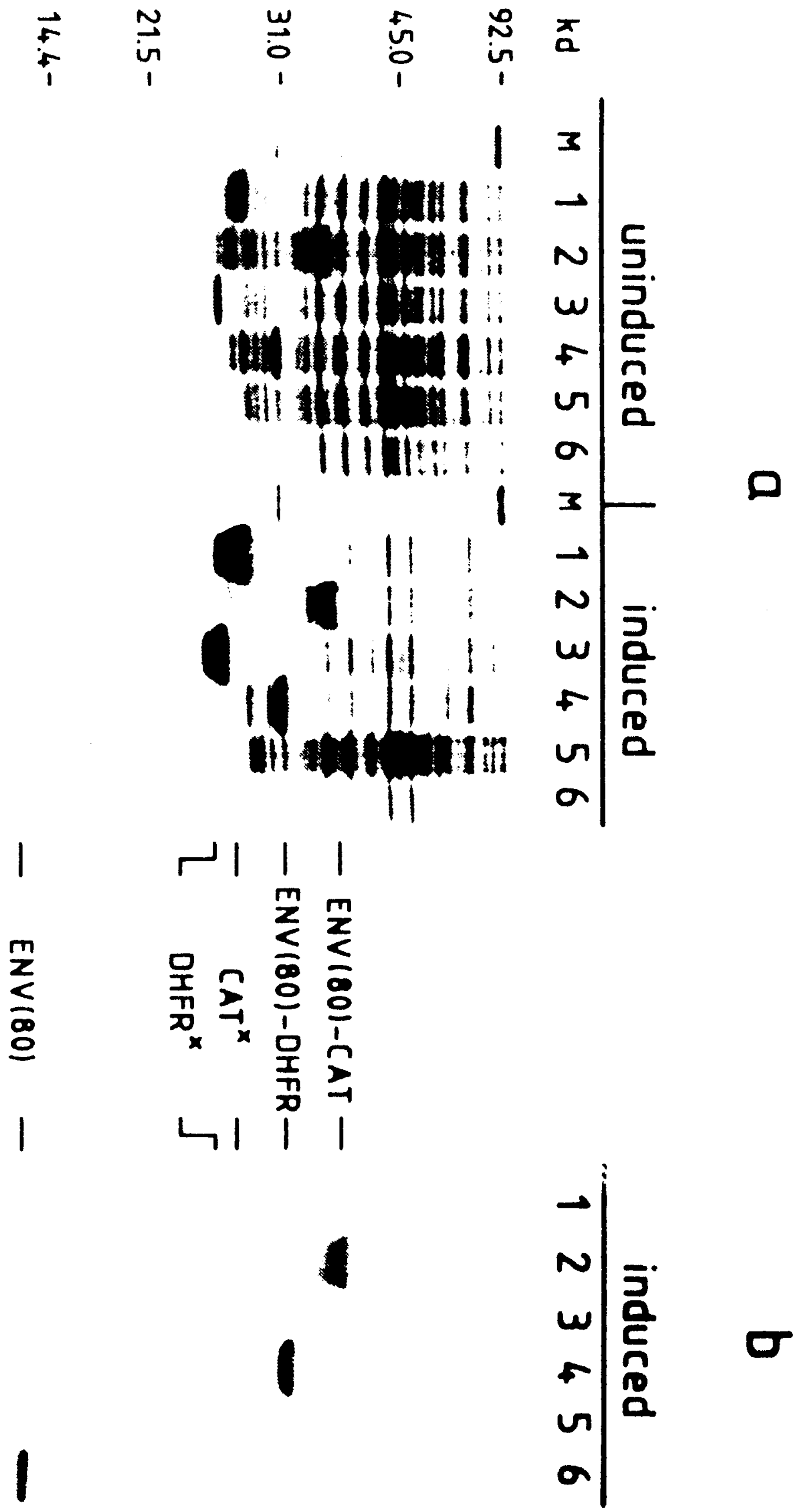

Fig. 10

0219106

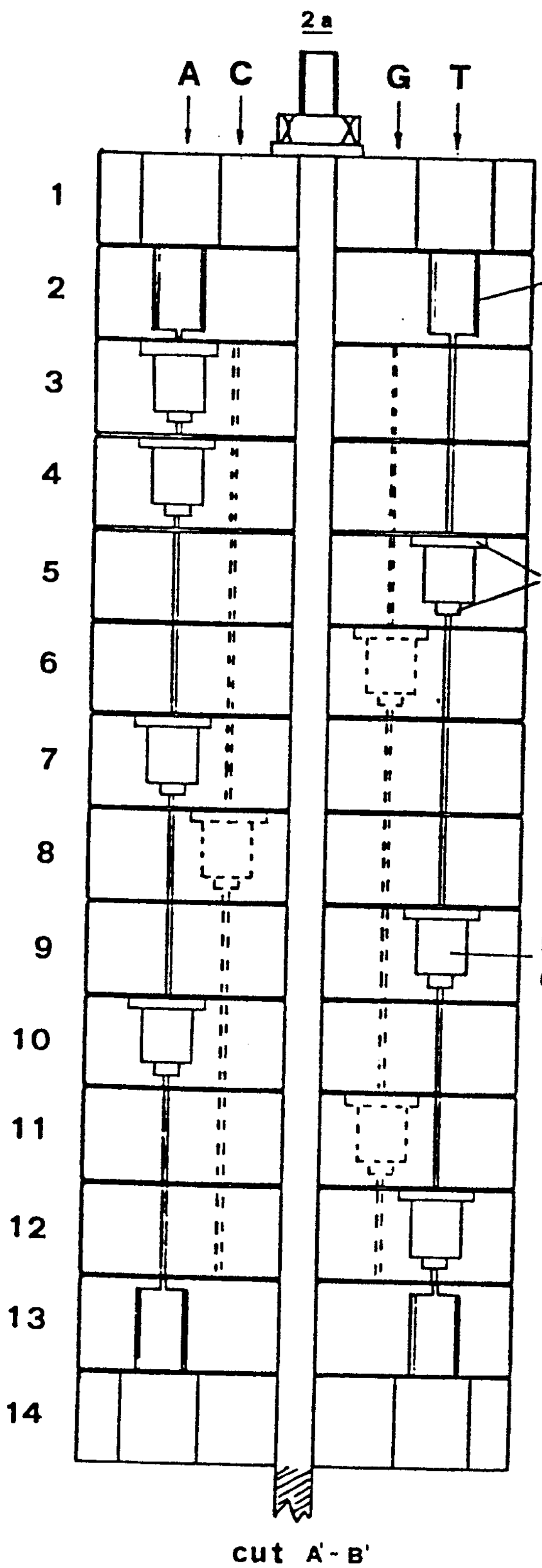


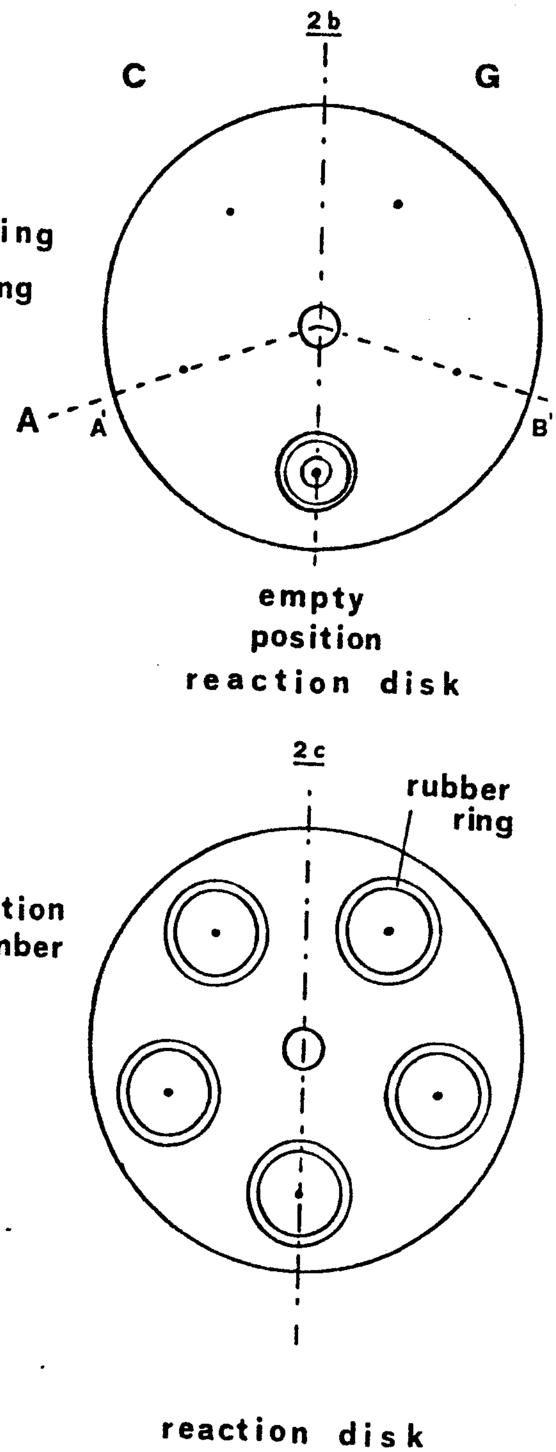


1, 14 metal disk

2, 13 disks with fitting for the tubing

3-12 reaction disks